# EUROPEAN PATENT APPLICATION

(11) **EP 3 530 758 A1**
(43) Date of publication of application: **28.08.2019**
(21) Application number: 18305190.3
(22) Date of filing: 23.02.2018
(51) Int. Cl.: C12Q 1/6886

(54) **METHOD FOR DETERMINING THE IN VITRO PRESENCE OF A TARGET MOLECULE IN A BIOLOGICAL SAMPLE, AND ITS USE FOR THE IN VITRO ANALYSIS OF CANCERS, IN PARTICULAR SOFT-TISSUE SARCOMAS AND GASTROINTESTINAL STROMAL TUMORS**

(71) Applicant: Université de Bordeaux, 33000 Bordeaux (FR); INSERM (Institut National de la Santé et de la Recherche Médicale), 75013 Paris (FR); Institut Bergonié, 33000 Bordeaux (FR); Université Paul Sabatier Toulouse III, 31400 Toulouse (FR); Institut Claudius Regaud, 31100 Toulouse (FR)
(72) Inventor: CHIBON, Frédéric, 31860 Labarthe-sur-Lèze (FR); LESLUYES, Tom, 31000 Toulouse (FR); LE GUELLEC, Sophie, 31000 Toulouse (FR)
(74) Representative: Novagraaf Technologies

(57) **Abstract**

The present invention relates to a method for determining *in vitro* the presence and/or the level of target molecules in a biological sample, comprising the steps of:
(1) placing said biological sample in the presence of a pool of different probes, each probe comprising (a) a binding region specific of one target molecule, and eventually (b) a detectable region that is unique for each of the at least one target molecule, wherein the binding region of a probe comprises a polynucleotide having a sequence selected in the group consisting of SEQ ID NO: 1 to SEQ ID NO: 67 or of SEQ ID NO: 1 to SEQ ID NO: 75, and wherein the presence of a target molecule in the biological sample allows the formation of a specific detectable molecular complexes between said probe and said target molecule, and
(2) detecting and/or counting the molecular complex(es) formed, thus allowing the determination of the presence and/or of the level of said at least one target molecule in the biological sample.

The present invention also relates to the use of the method for the *in vitro* analysis of a cancer.

The present invention also relates to a pool of polynucleotides comprising the polynucleotides of sequence SEQ ID NO : 1 to SEQ ID NO : 67 or SEQ ID NO : 1 to SEQ ID NO : 75, and to its use in a method for determining *in vitro* the presence and/or the level of at least one target molecule in a biological sample.

## Description

### Technical field

The present invention refers to a method for determining the *in vitro* presence and/or the level of target molecules in a biological sample, to the use of the method for the *in vitro* analysis of a cancer, and to a pool of polynucleotides usable in a method for determining *in vitro* the presence and/or the level of at least one target molecule in a biological sample.

Therefore, the present invention has utility in diagnostic, prognostic and therapeutic fields.

In the description below, the references into brackets ([ ]) refer to the listing of references situated at the end of the text.

### Background of the Invention

Adult soft-tissue sarcomas are rare tumors (<1%) that form a heterogeneous group with more than 100 different pathological subtypes and an aggressive clinical course (40/50% metastases within 5 years of diagnosis) (Fletcher CDM, Bridge JA, Hogendoorn PCW, et al. World Health Organization classification of tumours: Pathology and genetics of tumours of soft tissue and bone. Lyon, France: IARC Press 2012 ([**1**])). Clinical management consists in surgical resection with optional adjuvant therapies that depend on clinical characteristics, tumor histological type and histological grade (Trojani M, Contesso G, Coindre JM et al. Soft-tissue sarcomas of adults; study of pathological prognostic variables and definition of a histopathological grading system. Int J Cancer 1984;33:37e42 (**[2]**)). The histological grading system of the FNCLCC (*Fédération Nationale des Centres de Lutte Contre le Cancer*) comprises three grades: low, intermediate and high risk of metastasis. It is at present the best predictor of metastatic relapse in early-stage sarcoma and the most influential for deciding on adjuvant chemotherapy (Italiano A, Delva F, Mathoulin-Pelissier S et al. Effect of adjuvant chemotherapy on survival in FNCLCC grade 3 soft tissue sarcomas: a multivariate analysis of the French Sarcoma Group Database. Ann Oncol 2010; 21: 2436-2441 (**[3]**)). However, it has several limitations. It is not applicable in all pathological subtypes, its reproducibility may vary between pathologists, it is poorly informative for grade 2, representing ∼40% of cases, and it is difficult to apply on tumor microbiopsies (Coindre JM, Terrier P, Guillou L et al. Predictive value of grade for metastasis development in the main histologic types of adult soft tissue sarcomas: a study of 1240 patients from the French Federation of Cancer Centers Sarcoma Group. Cancer 2001;91:1914e26 **([4])).** In 2010, a 67-gene expression signature named CINSARC (Complexity Index in SARComas) was identified and validated as a valuable prognostic factor in several sarcoma histotypes and outperforms histological grade (Chibon F, Lagarde P, Salas S et al. Validated prediction of clinical outcome in sarcomas and multiple types of cancer on the basis of a gene expression signature related to genome complexity. Nat Med. 2010; 16:781-7 **([5]);** Lagarde P, Pérot G, Kauffmann A, et al. Mitotic checkpoints and chromosome instability are strong predictors of clinical outcome in gastrointestinal stromal tumors. Clin Cancer Res 2012;18:826e38 **([6])** ; Lagarde P, Przybyl J, Brulard C, et al. Chromosome instability accounts for reverse metastatic outcomes of pediatric and adult synovial sarcomas. J Clin Oncol 2013;31:608e15 **([7])).** CINSARC, in which genes are involved in mitotic control and chromosomal integrity, stratifies tumor prognosis into two groups ("low-risk, C1" and "high-risk, C2"), instead of three with the FNCLCC system, thus facilitating clinical management. It was first used on frozen tumors analyzed by microarrays and was subsequently applied on formalin-fixed, paraffin-embedded (FFPE) RNA sequencing (Lesluyes T, Pérot G, Largeau MR, et al. RNA sequencing validation of the Complexity INdex in SARComas prognostic signature. Eur J Cancer 2016; 22:104-111 **([8])).** However, the RNA is degraded in more than half of all tested FFPE tumors so its routine use for clinical and therapeutic purposes has been limited until now. Indeed, the availability of fresh-frozen tumor material is very low in daily routine practice. Archived FFPE tumor tissue samples are available in anatomical pathology laboratories and tissue banks. Unfortunately, cryopreservation induces chemical modifications such as crosslinking between nucleic acids and proteins and RNA degradation, so the subsequent use of frozen tissue for genomic and transcriptomic applications such as microarrays and RNA sequencing is limited (Von Ahlfen S, Missel A, Bendrat K, Schlumpberger M: Determinants of RNA quality from FFPE samples. PLoS One 2007, 2(12):e1261 **([9])).**

NanoString, a recently developed probe-based technique using direct digital measurement of transcript abundance with multiplexed color-coded probe pairs, has been shown to quantify mRNA expression accurately in FFPE and fragmented material (Geiss GK, Bumgarner RE, Birditt B, Dahl T, Dowidar N, et al. (2008) Direct multiplexed measurement of gene expression with color-coded probe pairs. Nat Biotechnol 26(3): 317-325 **([10]);** Malkov VA, Serikawa KA, Balantac N, Watters J, Geiss G, et al. (2009). Multiplexed measurements of gene signatures in different analytes using the Nanostring nCounter Assay System. BMC Res Notes 2: 80 **([11])).** NanoString is hybridization-based and does not require reverse transcription of mRNA and subsequent cDNA amplification. This feature of nanoString offers advantages compared to PCR-based methods, including the absence of amplification bias, which may be higher when using fragmented RNA isolated from FFPE blocks. For example, it was observed massive ERCC spike-in (quality control RNA) sequencing in FFPE blocks with strong RNA degradation due to the amplification step (Lesluyes *et al.* **([8])).** Several publications have demonstrated the accuracy and precision of nanoString with FFPE material (Geiss *et al.* **([10])** ; Northcott PA, Shih DJ, Remke M, Cho YJ, Kool M, et al. (2012) Rapid, reliable, and reproducible molecular sub-grouping of clinical medulloblastoma samples. Acta Neuropathol 123(4): 615-626 **([12]);** Reis PP, Waldron L, Goswami RS, Xu W, Xuan Y, Perez-Ordonez B, Gullane P, Irish J, Jurisica I, Kamel-Reid S (2011) mRNA transcript quantification in archival samples using multiplexed, color-coded probes. BMC Biotechnol 11:46 **([13]))** and its superiority to real-time quantitative PCR with RNA extracted from FFPE material (Reis *et al.* **([13])).**

Thus, a need exists of alternative clinical practice, and especially clinical practice adapted to be used in routine, to determine the risk of developing metastases and to guide personalized therapeutic management of patients with cancers. The present invention fulfills these and other needs.

### Description of the invention

The Applicant built a code set consisted of 67 probes derived from the 67 genes of the CINSARC signature, and named it "NanoCind" To compare the performance of RNA-seq and nanoString (NanoCind) techniques, he used expressions of various sarcomas using both techniques and compared predictive values based on CINSARC risk groups and clinical annotations. He also used nanoString (NanoCind) on FFPE blocks and matched frozen and FFPE samples to compare their level of agreement. Metastasis-free survivals and agreement values in classification groups were evaluated.

The Applicant now found that CINSARC strongly predicted metastatic outcome using both RNA-seq and nanoString (NanoCind) on frozen samples, with similar risk-group classifications. While more than 50% of FFPE blocks were not analyzable by RNA-seq owing to poor RNA quality, almost all samples were analyzable with nanoString. When similar (risk-group) classifications were measured with frozen tumors (RNA-seq) compared to FFPE blocks, the CINSARC signature was still a predictive factor of metastatic outcome with nanoString (NanoCind) on FFPE samples.

The Applicant also found that CINSARC is a material-independent prognostic signature for metastatic outcome in cancers, preferably sarcomas and outperforms histological grade. When coupled with nanoString, CINSARC, a 67-gene signature (NanoCind), is predictive of metastatic relapse on FFPE blocks and outperforms standard histology. Unlike RNA-seq, nanoString is significantly less influenced by the poor quality of RNA extracted from FFPE blocks. Advantageously, the CINSARC signature can potentially be used in combination with nanoString (NanoCind) in routine clinical practice on FFPE blocks to predict metastatic outcome and to guide personalized therapeutic management.

Accordingly, in a first aspect, the present invention relates to a method for determining *in vitro* the presence and/or the level of target molecules in a biological sample, comprising the steps of:
(1) placing said biological sample in the presence of a pool of different probes, each probe comprising (a) a binding region specific of one target molecule, and eventually (b) a detectable region that is unique for each of the at least one target molecule, wherein the binding region comprises a polynucleotide having a sequence selected in the group consisting of SEQ ID NO: 1 to SEQ ID NO: 67 or of SEQ ID NO: 1 to SEQ ID NO: 75, and wherein the presence of a target molecule in the biological sample allows the formation of a specific detectable molecular complex between said probe and said target molecule, and
(2) detecting and/or counting the molecular complex(es) formed, thus allowing the determination of the presence and/or of the level of target molecules in the biological sample.

"Target molecules" refers herein to the 67-gene expression signature named CINSARC (Complexity Index in SARComas). The target molecules are the 67 polynucleotide sequences of the 67 genes constituting this signature. The polynucleotide sequences may be preferably RNA, but can also be any polynucleotide sequence derived from RNA. The 67 genes of the CINSARC signature are given in Table 1 below:

**Table 1: Location and cytoband of 67 genes of the CINSARC signature**

| **Genes** | **Chromosome** | **Cytoband** |
|---|---|---|
| ANLN | 7 | p14.2 |
| ASPM | 1 | q31.3 |
| AURKA | 20 | q13.2 |
| AURKB | 17 | p13.1 |
| BIRC5 | 17 | q25.3 |
| BORA (also named C13orf34) | 13 | q22.1 |
| BUB1 | 2 | q13 |
| BUB1B | 15 | q15.1 |
| CCNA2 | 4 | q27 |
| CCNB1 | 5 | q13.2 |
| CCNB2 | 15 | q22.2 |
| CDC20 | 1 | p34.2 |
| CDC45 | 22 | q11.21 |
| CDC6 | 17 | q21.2 |
| CDC7 | 1 | p22.2 |
| CDCA2 | 8 | p21.2 |
| CDCA3 | 12 | p13.31 |
| CDCA8 | 1 | p34.3 |
| CDK1 (also named CDC2) | 10 | q21.2 |
| CENPA | 2 | p23.3 |
| CENPE | 4 | q24 |
| CENPL | 1 | q25.1 |
| CEP55 | 10 | q23.33 |
| CHEK1 | 11 | q24.2 |
| CKAP5 | 11 | p11.2 |
| CKS2 | 9 | q22.2 |
| ECT2 | 3 | q26.31 |
| ESPL1 | 12 | q13.13 |
| FANCI (also named KIAA1794) | 15 | q26.1 |
| FBXO5 | 6 | q25.2 |
| FOXM1 | 12 | p13.33 |
| H2AFX | 11 | q23.3 |
| HP1BP3 | 1 | p36.12 |
| KIF11 | 10 | q23.33 |
| KIF14 | 1 | q32.1 |
| KIF15 | 3 | p21.31 |
| KIF18A | 11 | p14.1 |
| KIF20A | 5 | q31.2 |
| KIF23 | 15 | q23 |
| KIF2C | 1 | p34.1 |
| KIF4A | X | q13.1 |
| MAD2L1 | 4 | q27 |
| MCM2 | 3 | q21.3 |
| MCM7 | 7 | q22.1 |
| MELK | 9 | p13.2 |
| NCAPH | 2 | q11.2 |
| NDE1 | 16 | p13.11 |
| NEK2 | 1 | q32.3 |
| NUF2 | 1 | q23.3 |
| OIP5 | 15 | q15.1 |
| PBK | 8 | p21.1 |
| PLK4 | 4 | q28.2 |
| PRC1 | 15 | q26.1 |
| PTTG1 | 5 | q33.3 |
| RAD51AP1 | 12 | p13.32 |
| RNASEH2A | 19 | p13.2 |
| RRM2 | 2 | p25.1 |
| SGO2 | 2 | q33.1 |
| SMC2 | 9 | q31.1 |
| SPAG5 | 17 | q11.2 |
| SPC25 (also named SPBC25) | 2 | q31.1 |
| TOP2A | 17 | q21.2 |
| TPX2 | 20 | q11.21 |
| TRIP13 | 5 | p15.33 |
| TTK | 6 | q14.1 |
| UBE2C | 20 | q13.12 |
| ZWINT | 10 | q21.1 |

In an embodiment of the invention, the group of target molecules may include the 8 additional genes that are used as housekeeping genes in the invention, and are mentioned in Table 2 below:

**Table 2: housekeeping genes**

| Gene | Chromosome | Cytoband |
|---|---|---|
| ACTB | 7 | p22.1 |
| B2M | 15 | q21.1 |
| GAPDH | 12 | p13.31 |
| HPRT1 | X | q26.2-q26.3 |
| PGK1 | X | q21.1 |
| PPIA | 7 | p13 |
| RPLPO | 12 | q24.23 |
| SDHA | 5 | p15.33 |

In the purposes of the invention, the expression of the housekeeping genes is used as a reference (for normalization purpose) point for the analysis of expression levels of the 67 genes constituting the CINSARC signature. Indeed, these are all well expressed genes in many tissue types, commonly used as household genes for transcriptomic studies.

"Biological sample" refers herein to any kind of sample likely to contain the target molecules. The biological sample may be derived from a tissue sample of a patient having a soft-tissue sarcomas (STS), a gastrointestinal stromal tumors (GIST) or other tumor types since CINSARC predicts aggressiveness in multiple neoplasms (Lesluyes T, Delespaul L, Coindre JM, Chibon F. The CINSARC signature as a prognostic marker for clinical outcome in multiple neoplasms. Sci Rep. 2017; 14;7(1):5480 **([14])).** For example, the tissue sample may be derived from (i) a primary tumor, (ii) from the center of a tumor, (iii) a site in the tumor other than the center or (iv) any tumor located outside the tumor tissue *per se,* this including metastases. The biological tumor sample may come for example from a surgical procedure, or from a tumor resection performed on the patient, a biopsy where a part of the tumor tissue is collected for later analysis, or from a blood sample, for example whole blood, plasma or serum, containing tumor cells derived from the primary tumor or tumor proteins produced by the tumor cells derived from the primary tumor. Preferably, the tissue sample comes from surgical resections of untreated primary tumors. The biological sample may be a fresh sample, or a frozen sample, or a formalin-fixed, paraffin-embedded (FFPE) block. Advantageously, total RNA can be extracted from paraffin-embedded tissue samples using commercially available kits such as ARN FFPET (Roche) or RecoverAll Total Nucleic Acid Isolation Kit (Ambion) following manufacturer's protocols..

"probe" refers herein to a polynucleotide sequence that is able to hybridize to one specific target molecule, *i.e.* to form a molecular complex with one specific target molecule. As one probe is specific to only one of the target molecules, each probe binds specifically to one of the 67 or 75 target molecules.

"pool of probes" refers herein to the group of 67 or 75 different probes, each probe being specific of only one target molecule. According to the invention, the pool "consists of" the entire group of 67 or 75 target molecules, meaning that the method of the invention uses, in step a), 67 or 75 different probes.

According to the invention, each probe can bind specifically to only one target molecule via its binding region. The binding region is a polynucleotide sequence that can hybridize, under suitable conditions, to one target molecule or at least a part of it. Advantageously, each binding region is complementary to at least a part of one specific target molecule, thus allowing the hybridization. The binding region can be formed exclusively of the hybridizable polynucleotide sequence, or may contain other polynucleotide sequence(s), such as sequence(s) allowing the attachment of the detectable region of the probe or be coupled with a short sequence linked to biotin..

The correspondence between the target molecules and the probes/binding regions are given in Table 3 below:

**Table 3:**

| **Target molecule** | **Sequence of the binding region of the probe** |
|---|---|
| ANLN | SEQ ID NO : 1 |
| ASPM | SEQ ID NO : 2 |
| AURKA | SEQ ID NO : 3 |
| AURKB | SEQ ID NO : 4 |
| BIRC5 | SEQ ID NO : 5 |
| BORA | SEQ ID NO : 6 |
| BUB1 | SEQ ID NO : 7 |
| BUB1B | SEQ ID NO : 8 |
| CCNA2 | SEQ ID NO : 9 |
| CCNB1 | SEQ ID NO : 10 |
| CCNB2 | SEQ ID NO : 11 |
| CDC20 | SEQ ID NO : 12 |
| CDC45 | SEQ ID NO : 13 |
| CDC6 | SEQ ID NO : 14 |
| CDC7 | SEQ ID NO : 15 |
| CDCA2 | SEQ ID NO : 16 |
| CDCA3 | SEQ ID NO : 17 |
| CDCA8 | SEQ ID NO : 18 |
| CDK1 | SEQ ID NO : 19 |
| CENPA | SEQ ID NO : 20 |
| CENPE | SEQ ID NO : 21 |
| CENPL | SEQ ID NO : 22 |
| CEP55 | SEQ ID NO : 23 |
| CHEK1 | SEQ ID NO : 24 |
| CKAP5 | SEQ ID NO : 25 |
| CKS2 | SEQ ID NO : 26 |
| ECT2 | SEQ ID NO : 27 |
| ESPL1 | SEQ ID NO : 28 |
| FANCI | SEQ ID NO : 29 |
| FBXO5 | SEQ ID NO : 30 |
| FOXM1 | SEQ ID NO : 31 |
| H2AFX | SEQ ID NO : 32 |
| HP1BP3 | SEQ ID NO : 33 |
| KIF11 | SEQ ID NO : 34 |
| KIF14 | SEQ ID NO : 35 |
| KIF15 | SEQ ID NO : 36 |
| KIF18A | SEQ ID NO : 37 |
| KIF20A | SEQ ID NO : 38 |
| KIF23 | SEQ ID NO : 39 |
| KIF2C | SEQ ID NO : 40 |
| KIF4A | SEQ ID NO : 41 |
| MAD2L1 | SEQ ID NO : 42 |
| MCM2 | SEQ ID NO : 43 |
| MCM7 | SEQ ID NO : 44 |
| MELK | SEQ ID NO : 45 |
| NCAPH | SEQ ID NO : 46 |
| NDE1 | SEQ ID NO : 47 |
| NEK2 | SEQ ID NO : 48 |
| NUF2 | SEQ ID NO : 49 |
| OIP5 | SEQ ID NO : 50 |
| PBK | SEQ ID NO : 51 |
| PLK4 | SEQ ID NO : 52 |
| PRC1 | SEQ ID NO : 53 |
| PTTG1 | SEQ ID NO : 54 |
| RAD51AP1 | SEQ ID NO : 55 |
| RNASEH2A | SEQ ID NO : 56 |
| RRM2 | SEQ ID NO : 57 |
| SGO2 | SEQ ID NO : 58 |
| SMC2 | SEQ ID NO : 59 |
| SPAG5 | SEQ ID NO : 60 |
| SPC25 | SEQ ID NO : 61 |
| TOP2A | SEQ ID NO : 62 |
| TPX2 | SEQ ID NO : 63 |
| TRIP13 | SEQ ID NO : 64 |
| TTK | SEQ ID NO : 65 |
| UBE2C | SEQ ID NO : 66 |
| ZWINT | SEQ ID NO : 67 |
| ACTB | SEQ ID NO : 68 |
| B2M | SEQ ID NO : 69 |
| GAPDH | SEQ ID NO : 70 |
| HPRT1 | SEQ ID NO : 71 |
| PGK1 | SEQ ID NO : 72 |
| PPIA | SEQ ID NO : 73 |
| RPLP0 | SEQ ID NO : 74 |
| SDHA | SEQ ID NO : 75 |

Suitable conditions allowing the hybridization of a target molecule and the corresponding binding region of a probe are standard conditions that can be determined experimentally by the skilled person without undue burden.

According to the invention, each probe may eventually contain a detectable region that is unique for each target molecule. In an embodiment of the invention, the detectable region may be located on a second probe, which is then a reporter probe. This second probe may consist of a second 50mer target-specific oligonucleotide linked to a unique chain of dye-labelled segments for detection by the system. In tis case, unique multiplexed probes were made with two sequence-specific probes complementary to a 100-base target region per target mRNA. This technique may be performed for example as described by Geiss *et al.* **([10]).** The detectable region may be detected by any means routinely used by the skilled person in the field of detection of hybridization probes. The detectable region may comprise for example a detectable signal chosen among fluorescence, luminescence, colorimetry and a magnetic field. For example, the detectable region may comprise one or more fluorochrome(s) defining a unique detectable signature. The detectable region may eventually be designed as described in document WO2010/019826 **([18]).**

Advantageously, if the binding region binds to a target molecule present in the biological sample, the detectable region generates an unique signal that does not change during the course of a detection assay, and so remains the same during the course of a detection assay.

The detection and/or counting of the molecular complexes of step (2) may be performed using any suitable method known in the art. For example, if the detectable signal is a luminescent signal, the brightness of a signal may allow to determine the strength, size and/or intensity of the signal. The signal may then be analysed using standard image analysis software/algorithm or user to define what a spot of a particular color should look like in term of brightness and size. In one embodiment, step (2) comprises an individual counting of the detectable region of the probe, or a general counting.

In a general manner, step (1) and/or (2) of the method of the invention may include all or part of Nanostring protocol, notably as defined in anyone of documents Geiss *et al.* **([10]),** Malkov *et al.*(**[11]**))*,* , Northcott *et al.* **([12]),** Reis *et al.* (**[13]**) and WO02010/019826 (**[18]**).

Another object of the present invention relates to the use of the method of the invention for the *in vitro* analysis of a cancer. As mentioned in document WO2010/122243 **([19]),** the 67 genes of the CINSARC signature are involved in the same biological process, *i.e.* the chromosomal integrity. Without wanting to be bound by a theory, it is assumed that this explains that the CINSARC signature can be used to predict tumor aggressiveness in different types of cancers, such as: breast, lung, colon, ovarian, hematopoietic and others described by Lesluyes *et al.* (**[14]**). Preferably, the cancer is chosen among soft-tissue sarcomas (STS) and gastrointestinal stromal tumors (GIST).

Interestingly, the method of the invention may be used for predicting, in said pathology, the occurrence of metastases, or for distinguishing subgroups of patients of good or bad prognosis. For the purpose of the present invention, the term "good prognosis" refers to the indication of patients who are not likely to relapse, that is to say the occurrence of metastases, during their treatment or in the following 5 to 6 years following their treatment. Thus in the context of the present invention; patients may be considered to belong to a "good prognosis" subgroup when their CINSARC signature correlates with the good prognosis reference group and are prone to develop metastases in less than 20% of cases all types of cancer, especially sarcomas. On the other hand, the term "poor prognosis" refers to patients who may have a relapse (metastases) during treatment or within 5 to 6 years of treatment. Thus, in the context of the present invention, patients may be considered to belong to a subgroup of poor prognosis when their CINSARC signature correlates with the poor prognosis reference group and are subject to develop metastases at a high frequency. This may be done for example by a method as disclosed in anyone of documents Chibon *et al.* **([5]),** Lagarde *et al.* (**[6]**), Lagarde *et al.* **([7]),** Lesluyes *et al.* **([8]),** Lesluyes *et al.* **([14])** and WO2010/122243 **([19]).** For example, it is possible, after step (2) as described above, to compare the level of expression obtained in step (2) with the level of expression of the same polynucleotide pool measured in a biological control group, a deregulation of the level of expression of the polynucleotide pool with respect to its level of expression corresponding measured in a biological control group being predictive of the occurrence of metastasis, and/or allowing to identify a subgroup of patients of good prognosis or a subgroup of patients of poor prognosis. The biological control group may be tissue samples derived from tumors of other patients suffering from the same cancer than that to be tested, the grade of which is previously known, Thus the tumors can be classified with respect to each other, depending on the level of expression of the genes of the molecular signature of the invention in each case.

Another object of the invention relates to a polynucleotide comprising a sequence chosen among SEQ ID NO : 1 to SEQ ID NO : 75.

Another object of the invention relates to a pool of polynucleotides comprising the polynucleotides of sequence SEQ ID NO : 1 to SEQ ID NO : 67. The pool of the invention may also contain in addition the polynucleotides of sequence SEQ ID NO : 68 to SEQ ID NO : 75. The pool may comprise 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74 or 75 polynucleotides chosen among SEQ ID NO : 1 a SEQ ID NO : 75.

In one embodiment, the pool consists of a group of polynucleotides of sequence SEQ ID NO : 1 to SEQ ID NO : 67 or SEQ ID NO : 1 to SEQ ID NO : 75. In these embodiments, the groups contain respectively 67 and 75 polynucleotides.

Another object of the invention is the use of a pool of polynucleotides of the invention, in a method for determining *in vitro* the presence and/or the level of at least one target molecule in a biological sample.

Another objet of the invention relates to a kit comprising:
- a pool of probes as defined in claim 1, capable of specifically forming a molecular complex with a target molecule, and
- means for detecting and/or quantifying said molecular complex.

According to the invention, the pool of probes may be localized on a suitable support, such as a RNA chip. The kit may further comprise the means involved in the hybridization of the target molecules and the pool of probes, such as hybridization medium.

The kit may be used for *in vitro* predicting the occurrence of metastases, and/or for distinguishing subgroups of good or bad prognosis in patients having a cancer, such as soft-tissue sarcomas (STS), gastrointestinal stromal tumors (GIST) or other cancers as described above.

This invention is further illustrated by the following examples with regard to the annexed drawings that should not be construed as limiting.

### Brief description of the figures

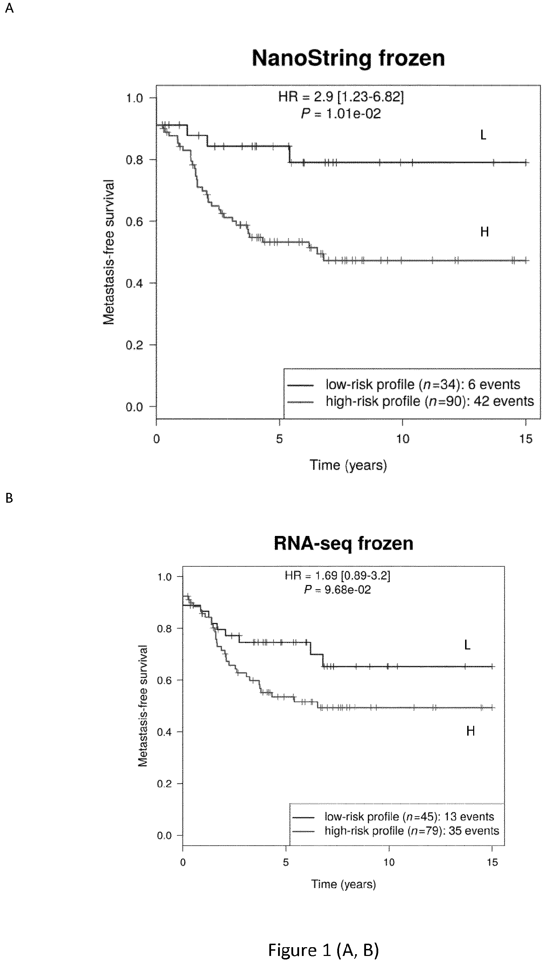

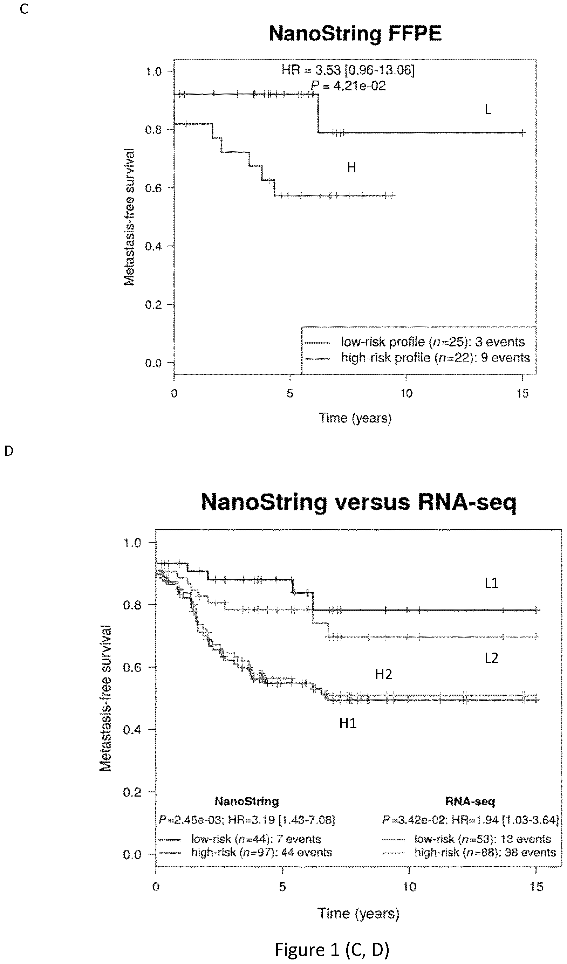

- Figure 1: represents the metastasis-free survival analysis according to CINSARC signature. (A) Prognostic value of nanoString expression data (cohort #1, fresh-frozen samples). L= low-risk profile (n=34), 6 events. H= high-risk profile (n=90), 42 events. (B) Prognostic value of RNA-seq expression data (cohort #1, fresh-frozen samples). L= low-risk profile (n=45), 13 events. H= high-risk profile (n=79), 35 events). (C) Prognostic value of nanoString expression data (NanoCind), (cohort #2, FFPE samples). L= low-risk profile (n=25), 3 events. H= high-risk profile (n=22), 9 events. (D) Comparison of prognostic values of the two techniques (1/ Nanostring and 2/ RNA-seq). Fresh-frozen and FFPE samples used with each technique were pooled. L1= low-risk profile (n=44), 7 events. H1= high-risk profile (n=97), 44 events. L2= low-risk profile (n=53), 13 events. H2= high-risk profile (n=88), 38 events.

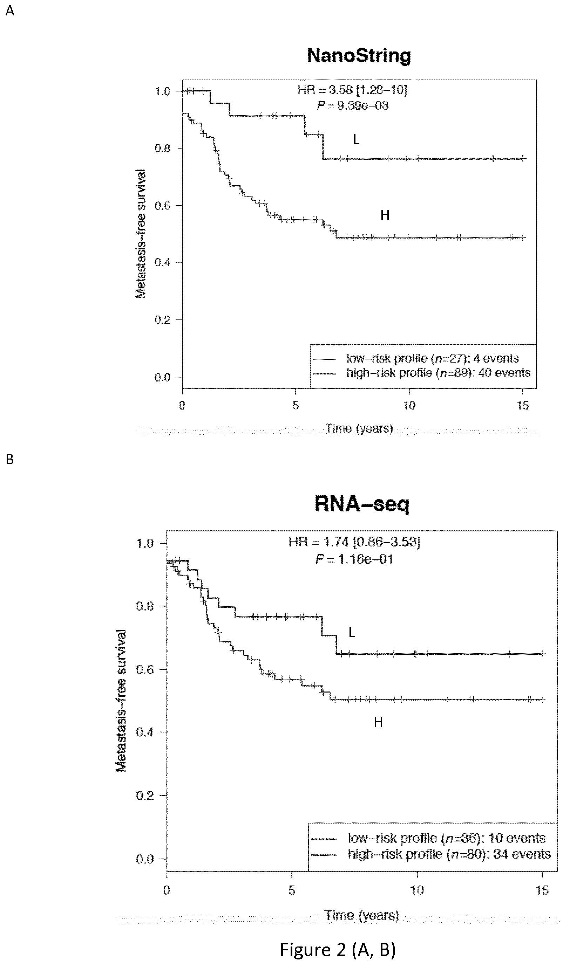

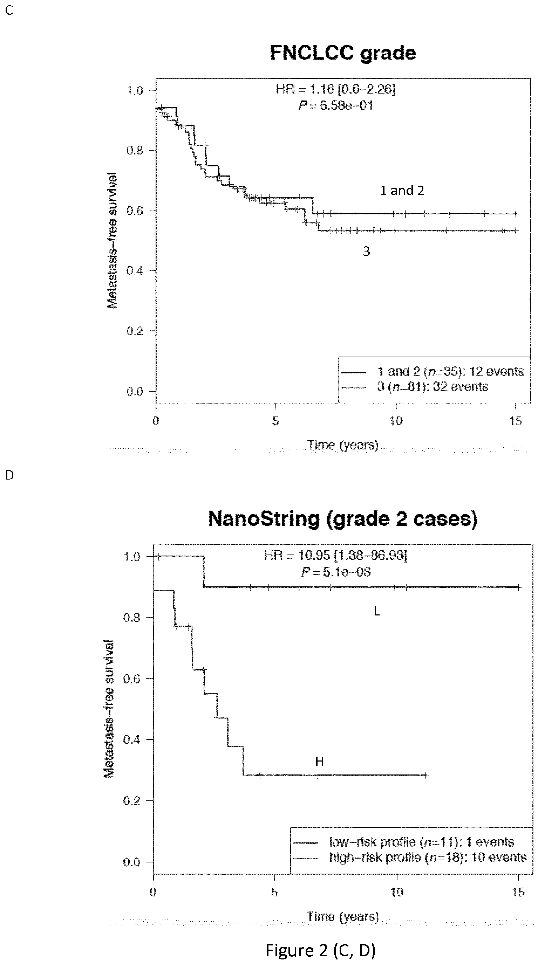

- Figure 2: represents the metastasis-free survival analysis (fresh-frozen samples, cohort #1 and FFPE samples, cohort #2) (A) according to CINSARC signature using nanoString expression data (NanoCind). L= low-risk profile (n=27), 4 events. H= high-risk profile (n=89), 40 events. (B) According to CINSARC signature using RNA-seq expression data. L= low-risk profile (n=10), 4 events. H= high-risk profile (n=89), 34 events. (C) According to FNCLCC grade. 1 and 2 (n=35), 12 events. 3 (n=81), 32 events. (D) Metastasis-free survival analysis according to CINSARC signature using nanoString expression data (NanoCind) on grade 2 FNCLCC cases. L= low-risk profile (n=11), 1 event. H= high-risk profile (n=18), 10 events.

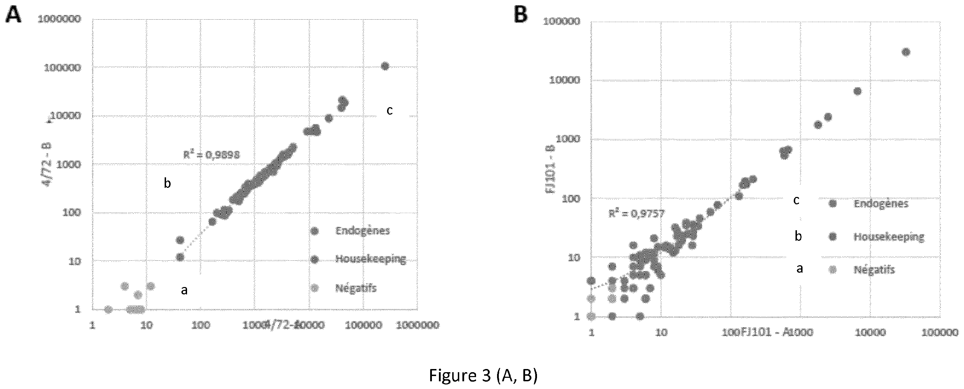

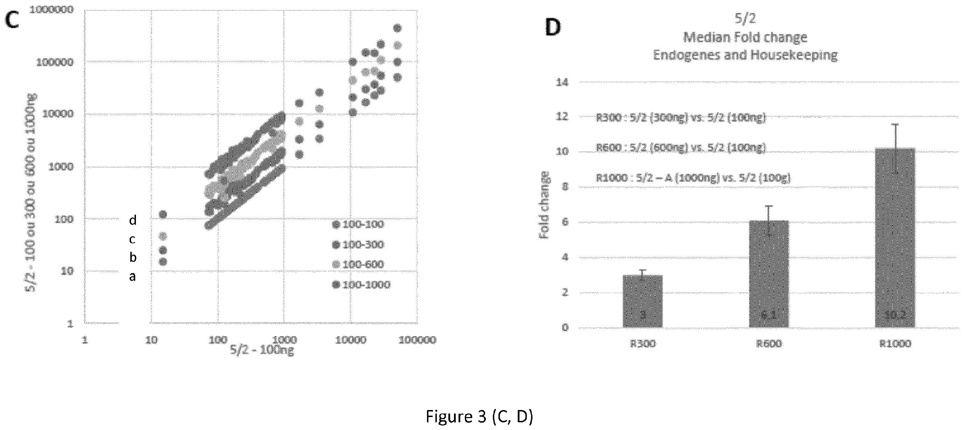

- Figure 3: represents the reproducibility and differential gene expression plots of sarcom 75 panel on the nanoString platform for two fresh frozen samples (100ng of total RNA). (A) Scatter plot of non-normalized signal for all 75 genes assayed is shown in log scale for technical replicates between two different cartridges. The R² value of a linear fit to this data is 0.989. a= negatives; b= housekeeping genes; c= endogens. (B) Scatter plot of non-normalized signal for all 75 genes assayed is shown in log scale for technical replicates in the same cartridge. The R² value of a linear fit to this data is 0.976. a= negatives; b= housekeeping genes; c= endogens. (C) Scatter plot of non-normalized signal for all 75 genes assayed is shown in log scale for technical replicates with different inputs (100ng (reference input, = a), 300ng (=b), 600ng (=c) and 1000ng (=d)) of RNA from the same fresh-frozen sarcoma samples. (D) Histogram established on normalized counts shows the median fold change (3, 6.1 and 10.2) induced by the increase in inputs, for R300 (300ng/100ng), R600 (600ng/100ng), R1000 (1000ng/100ng).

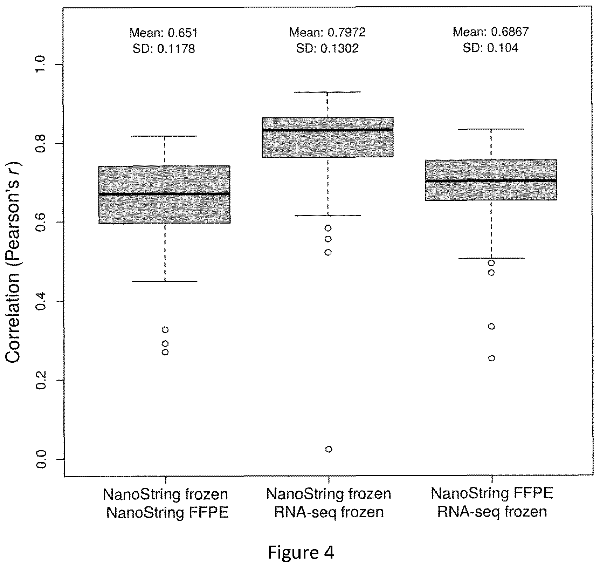

- Figure 4: represents raw comparison of CINSARC gene expression between RNA-seq and nanoString and in different materials (fresh-frozen and FFPE). (A) Boxplot of correlations obtained in cohort #1 between nanoString on fresh-frozen samples and nanoString on FFPE samples. (B) Boxplot of correlations obtained in cohorts #1 and #2 between RNA-seq on fresh-frozen samples (reference) and nanoString on fresh-frozen samples. (C) Boxplot of correlations obtained in cohort #1 between RNA-seq on fresh-frozen samples (reference) and nanoString on FFPE samples.

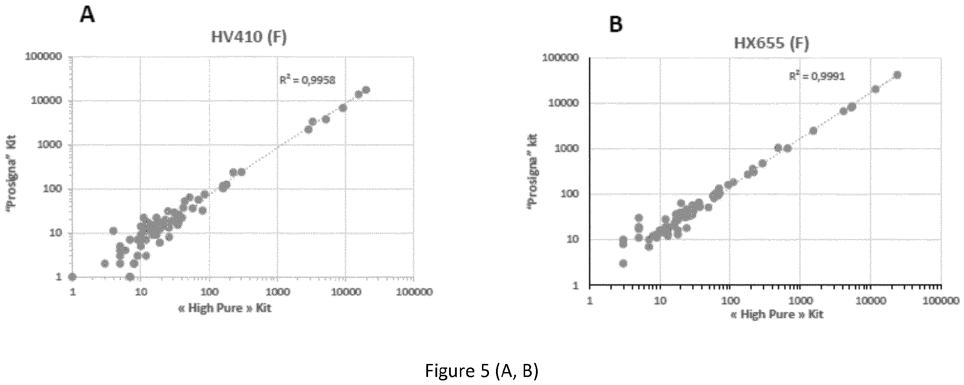

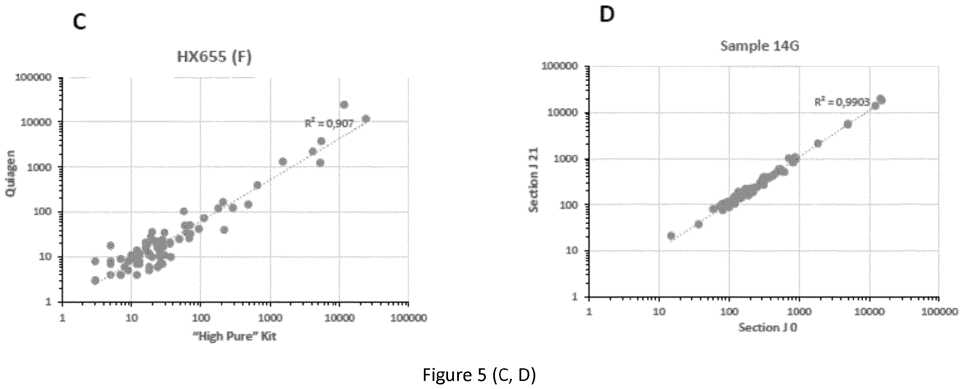

- Figure 5: represents Correlation of results obtained from nanoString analysis of same FFPE samples in different conditions. (A, B and C) Scatter plot of non-normalized signal for sarcom 75 panel in log-log scale with different extraction kits, A and B: High-pure kit/Prosigna kit; C: High-pure kit/ RNeasy FFPE kit. (D) Scatter plot of non-normalized signal for sarcom 75 panel in log-log scale for two different Extraction times: J0: RNA extraction performed on unstained slides the same day of the cut; J21: cut of unstained slides at J0 (stored at room temperature) and RNA extraction at J21. High-pure kit: High Pure FFPET RNA Isolation Kit (Roche); Prosigna kit, Roche FFPET RNA Isolation Kit, used in Prosigna process.

### Examples

### Example 1: Comparison of the performance of RNA-seq and nanoString in establishing CINSARC-based risk

To compare the performance of RNA-seq and nanoString in establishing CINSARC-based risk, we applied both techniques on sarcoma cohorts with clinical annotations from the Conticabase (European sarcoma database). We tested a large cohort of 124 frozen samples and a subgroup of 47 samples together with matching FFPE blocks to test the performance of each technique on degraded material typical of that used in daily practice.

### MATERIALS AND METHODS

### Cohorts and design

To evaluate the potential transfer of the CINSARC signature from RNA-seq on fresh-frozen tissues to nanoString on FFPE blocks, we used two independent cohorts. The first one which comprised 95 sarcomas was used in 2016 by Chibon's team to validate the transfer of CINSARC from microarrays to RNA-seq on fresh-frozen tissue (Lesluyes *et al.* **([8])).** Of the 95 sarcomas described in that publication, fresh-frozen tissues or RNA were still available for 77 cases. A second set of 47 sarcomas was added since RNA-seq expression on frozen tissues (n=47) and FFPE (n=41) had already been tested by our team. Therefore, for the transfer from RNA-seq to nanoString, we analyzed a set of 124 (77 and 47) sarcomas (named cohort #1) by both RNA-seq and nanoString (NanoCind) on fresh-frozen tissues. To evaluate whether CINSARC had a prognostic value on FFPE tissue, we analyzed the 47 sarcomas of cohort #2 with NanoCind on FFPE blocks.

### Tumor samples

Tumors were operated in expert centers of the French Sarcoma Group and came from surgical resections of untreated primary tumors. RNA was extracted from all FFPE blocks (n=47) in cohort #2 using the High Pure FFPET RNA Isolation Kit (Roche). To optimize the extraction protocol, we tested a second extraction kit (Roche FFPET RNA Isolation Kit, used in Prosigna Breast Cancer Prognostic Gene Signature). Finally, Lesluyes and al. **([8])** used the RNeasy FFPE kit (Qiagen) in 2016 for RNA extraction in 41 cases in cohort #2, but only 17 were analyzable by RNA-seq, owing to poor RNA quality as a result of formalin fixation.

### NanoString CodeSet design and expression quantification

This technique has already been described by Geiss et al. ([10]). Briefly, unique multiplexed probes were made with two sequence-specific probes complementary to a 100-base target region per target mRNA. The capture probe comprised a target-specific oligonucleotide coupled with a short sequence linked to biotin. The reporter probe consisted of a second 50mer target-specific oligonucleotide linked to a unique chain of dye-labelled segments for detection by the system. Our nCounter code set (sarcoma75) consisted of a panel of 75 probes, including 67 distinct test probes derived from 67 distinct genes (Table 4) and eight housekeeping genes for biological normalization purposes. We used 100 ng of total RNA isolated from fresh-frozen tissues, as suggested by the manufacturer. FFPE tissues required a higher amount of total RNA, corresponding to "100 ng adjusted" (adjusted input) according to the DV50-300 value, i.e. the percentage of RNA fragments between 50-300 nucleotides. Cases were assessed in cartridges of 12 cases per run. Data were extracted and validated using the nSolver v3.0 software.

**Table 4:**

| **Customer Identifier** | **Accession** | **Position** | **Target Sequence** |
|---|---|---|---|
| ACTB | NM_001101.2 | 1011-1110 | TGCAGAAGGAGATCACTGCCCTGGCACCCAGCACAATGAAGATCAAGATCA TTGCTCCTCCTGAGCGCAAGTACTCCGTGTGGATCGGCGGCTCCATCCT |
| ANLN | NM_018685.2 | 1901-2000 | ATGACCTCTTCAGTGATGTCCTAGAGGAAGGTGAACTAGATATGGAGAAGA GCCAAGAGGAGATGGATCAAGCATTAGCAGAAAGCAGCGAAGAACAGGA |
| ASPM | NM_ 018136.4 | 9361-9460 | TGATCCAAGCACATTATAGAGGATATAAAGGAAGGCAGGTCTTCTTCGGCA GAAATCTGCTGCTTTGATCATACAAAAATATATACGAGCCAGGGAGGC |
| AURKA | NM_ 003600.2 | 406-505 | AGCTCCAGTTGGAGGTCCAAAACGTGTTCTCGTGACTCAGCAATTTCCTTGT CAGAATCCATTACCTGTAAATAGTGGCCAGGCTCAGCGGGTCTTGTGT |
| AURKB | NM_004217.2 | 616-715 | AGATGCTCTAATGTACTGCCATGGAAGAAGGTGATTCACAGAGACATAAA GCCAGAAAATCTGCTCTTAGGGCTCAAGGAGAGCTGAAGATTGCTGAC |
| B2M | NM_004048.2 | 26-125 | CGGGCATTCCTGAAGCTGACAGCATTCGGGCCGAGATGTCTCGCTCCGTG GCCTTAGCTGTGCTCGCGCTACTCTCTCTTTCTGGCCTGGAGGCTATCCA |
| BIRC5 | NM_001168.2 | 1216-1315 | CCATTCTAAGTCATTGGGGAAACGGGGTGAACTTCAGGTGGATGAGGAGAC AGAATAGAGTGATAGGAAGCGTCTGGCAGATACTCCTTTTGCCACTGCT |
| BORA | NM_024808.2 | 653-752 | ATTTGCAGATCAATCTCCTGGAAACCTCAGTTCTTCATCCCTCAGAAGAAAGC |
| | | | TGTTTTAGATGGGAACGGAAGCATCTCCGACTCCTTACCTTCGGCT |
| BUB1 | NM_004336.3 | 1979-2078 | GAAGATAAAGAAAATGTGGTAGCAAAACAGTGTACCCAGGCGACTTTGGATT CTTGTGAGGAAAACATGGTGGTGCCTTCAAGGGATGGAAAATTCAGTC |
| BUB1B | NM_001211.4 | 836-935 | GAGTCTTCTGTACCACAACGAAGCACACTAGCTGAACTAAAGAGCAAAGGGA AAAAGACAGCAAGAGCTCCAATCATCCGTGTAGGAGGTGCTCTCAAGG |
| CCNA2 | NM_001237.2 | 1211-1310 | CGGGACAAAGCTGGCCTGAATCATTAATACGAAAGACTGGATATACCCTGGA AAGTCTTAAGCCTTGTCTCATGGACCTTCACCAGACCTACCTCAAAGC |
| CCNB1 | NM_031966.2 | 716-815 | AACTTGAGGAAGAGCAAGCAGTCAGACCAAAATACCTACTGGGTCGGGAAGT CACTGGAAACATGAGAGCCATCCTAATTGACTGGCTAGTACAGGTTCA |
| CCNB2 | NM_004701.2 | 981-1080 | AGGTTGATGTTGAACAGCACACTTTAGCCAAGTATTTGATGGAGCTGACTCTC ATCGACTATGATATGGTGCATTATCATCCTTCTAAGGTAGCAGCAGC |
| CDC20 | NM_001255.2 | 431-530 | GGAACATCAGAAAGCCTGGGCTTTGAACCTGAACGGTTTTGATGTAGAGGA AGCCAAGATCCTTCGGCTCAGTGGAAAACCACAAAATGCGCCAGAGGGT |
| CDC45 | NM_003504.3 | 1676-1775 | AGCTCCCGGATGCTGCACAACCATTTTGACCTCTCAGTAATTGAGCTGAAAG CTGAGGATCGGAGCAAGTTTCTGGACGCACTTATTTCCCTCCTGTCCT |
| CDC6 | NM_001254.3 | 1656-1755 | GGGGAAGTTATATGAAGCCTACAGTAAAGTCTGTCGCAAACAGCAGGTGG CGGCTGTGGACCAGTCAGAGTGTTTGTCACTTTCAGGGCTCTTGGAAGCC |
| CDC7 | NM_003503.2 | 806-905 | ATCCCACATAATCACAGGAAACAAGATTCCACTGAGTGGCCCAGTACCTAA GGAGCTGGATCAGCAGTCCACCACAAAAGCTTCTGTTAAAAGACCCTAC |
| CDCA2 | NM_152562.2 | 1751-1850 | AGACTTCAGTGGTCTCAGTTCCCTGCTGCTTGAGCAGTCACCTGTTCCTGA GCCATTACCTCAACCAGATTTTGATGACAAGGGGGAGAATCTTGAAAAC |
| CDCA3 | NM_031299.6 | 909-1008 | GCCTACCAGCAGGGGAGCAACTGGAGGGTCTTAAACATGCCCAGGACTCA GATCCCCGCTCTCCTACTCTTGGTATTGCACGGACACCTATGAAGACCAG |
| CDCA8 | NM_018101.2 | 1666-1765 | TCCCTGTTTACTGAAGACCAAATACTGGTTTGGAGACAACTTCCATGTCTTG CTCTTCTACCTCCCTAGTTAGTGGAAATTTGGATAAGGGAACTGTAGG |
| CDK1 | NM_001786.4 | 179-278 | GGTACCTATGGAGTTGTGTATAAGGGTAGACACAAAACTACAGGTCAAGTGG TAGCCATGAAAAAAATCAGACTAGAAAGTGAAGAGGAAGGGGTTCCTA |
| CENPA | NM_001042426.1 | 980-1079 | CACTTTGAGCAGTTGCCTGGAAGGCTGGGCATTTCCATCATATAGACCTCTG CCCTTCAGAGTAGCCTCACCATTAGTGGCAGCATCATGTAACTGAGTG |
| CENPE | NM_ 001813.2 | 292-391 | GCACCAATCATCGATTCTGCCATACAAGGCTACAATGGTACTATATTTGCCTA TGGACAGACTGCTTCAGGAAAAACATATACCATGATGGGTTCAGAAG |
| CENPL | NM_ 001127181.2 | 1441-1540 | GTGGCTACTACTGAATTTCTTTGGTCTGTACCCTGTAGCCCTCAAAGTCTGGA CATTTCTTTCGCAATACATCCAGAGGATGCAAAAGCTCTATGGGACA |
| CEP55 | NM_018131.3 | 571-670 | GTACTACCGCATTGCTTGAACAGCTGGAAGAGACAACGAGAGAAGGAGAAAG GAGGGAGCAGGTGTTGAAAGCCTTATCTGAAGAGAAAGACGTATTGAA |
| CHEK1 | NM_ 001114121.1 | 2226-2325 | AGGGTGATGGATTGGAGTTCAAGAGACACTTCCTGAAGATTAAAGGAAGCT GATTGATATTGTGAGCAGCCAGAAGATTTGGCTTCCTGCCACATGATC |
| CKAP5 | NM_014756.2 | 1416-1515 | TTGCTAAAGCCCTTTTGTGCTGCACTACTTAAGCACATCAATGATTCTGCTCCT |
| | | | GAAGTCAGAGATGCCGCATTTGAAGCATTGGGTACTGCTTTGAAGG |
| CKS2 | NM_ 001827.1 | 196-295 | AAACTCATCTGATGTCTGAAGAGGAGTGGAGGAGACTTGGTGTCCAACAGAGT CTAGGCTGGGTTCATTACATGATTCATGAGCCAGAACCACATATTCT |
| ECT2 | NM_018098.4 | 1361-1460 | CAGTTATTTCAAGTACCATTGGAAGAGGAAGGACAACGTGGTGGACCTATCCT TGCACCAGAGGAGATTAAGACTATTTTGGTAGCATCCCAGATATCT |
| ESPL1 | NM_012291.4 | 1286-1385 | CAGGGACTTCACCTCTACACTGTGGTGGTTTATGACTTTGCCCAAGGCTGTCA GATAGTTGATTTGGCTGACCTGACCCAACTAGTGGACAGTTGTAAAT |
| FANCI | NM_001113378.1 | 542-641 | TGAGTGGGGAAGAATGTAAGAAACAGTTGATTAACACCCTGTGTTCTGGCAGG TGGGATCAGCAATATGTAATCCAACTCACCTCCATGTTCAAGGATGT |
| FBXO5 | NM_012177.3 | 899-998 | GCACAACTCAGTGACATGGACTTAATCAATGTGTCTAAAGTGAGCACAACT TGGAAGAAGATCCTAGAAGATGATAAGGGGGCATTCCAGTTGTACAGTA |
| FOXM1 | NM_202002.1 | 1001-1100 | CAATTCGCCATCAACAGCACTGAGAGGAAGCGCATGACTTTGAAAGACATC TATACGTGGATTGAGGACCACTTTCCCTACTTTAAGCACATTGCCAAGC |
| GAPDH | NM_002046.3 | 973-1072 | CACTCCTCCACCTTTGACGCTGGGGCTGGCATTGCCCTCAACGACCACTTT GTCAAGCTCATTTCCTGGTATGACAACGAATTTGGCTACAGCAACAGGG |
| H2AFX | NM_002105.2 | 1393-1492 | CCGCCCCATTTCCCTTCCAGCAAACTCAACTCGGCAATCCAAGCACCTAGA TACCAGCACAAGTCGGTTAATCCCTGTCTGGACTGAGCCTCCGTTGGCT |
| HP1BP3 | NM_016287.3 | 307-406 | AGCTTGCTGAAGGGGAGGAAGAAAAGCCAGAACCAGACATAAGTTCAGAG GAATCTGTCTCCACTGTAGAAGAACAAGAGAATGAAACTCCACCTGCTAC |
| HPRT1 | NM_000194.1 | 241-340 | TGTGATGAAGGAGATGGGAGGCCATCACATTGTAGCCCTCTGTGTGCTCAA GGGGGGCTATAAATTCTTTGCTGACCTGCTGGATTACATCAAAGCACTG |
| KIF11 | NM_004523.3 | 383-482 | CAGACCATTTAATTTGGCAGAGCGGAAAGCTAGCGCCCATTCAATAGTAGAA TGTGATCCTGTACGAAAAGAAGTTAGTGTACGAACTGGAGGATTGGCT |
| KIF14 | NM_014875.2 | 4336-4435 | TGATCGAGCAATCCAGTCACTTACTATTCAGACTGCATGTGCTTTTGAGCAG CTAGTAGTGCTAATGAAACACTGGCTGAGTGATTTACTGCCTTGTACC |
| KIF15 | NM_020242.2 | 1531-1630 | AATTCCGAGAGGATCAAATAATACGCTTGGAAAAGCTCCACAAGGAATCCCG GGGAGGTTTTCTGCCTGAGGAGCAGGATCGTTTGCTCTCAGAATTAAG |
| KIF18A | NM_031217.3 | 1626-1725 | CATAGACTTGCAATGTTGAAAACTCGTCGCTCCTACCTGGAGAAAAGGAGGG AGGAGGAATTGAAGCAATTTGATGAGAATACTAATTGGCTCCATCGTG |
| KIF20A | NM_005733.2 | 1210-1309 | CACTTCCTTGAAGAGGAGTGTCTACATCGAAAGTCGGATAGGTACCAGCACC AGCTTCGACAGTGGCATTGCTGGGCTCTCTTCTATCAGTCAGTGTACC |
| KIF23 | NM_138555.1 | 3036-3135 | TGTGTGGATGATTTCTCGAAAGCCATGCCAGAAGCAGTCTTCCAGGTCATCT TGTAGAACTCCAGCTTTGTTGAAAATCACGGACCTCAGCTACATCATA |
| KIF2C | NM_006845.3 | 1941-2040 | CCTGCTCTAACGGGGCGCTGATTCCAGGCAATTTATCCAAGGAAGAGGAGG AACTGTCTTCCCAGATGTCCAGCTTTAACGAAGCCATGACTCAGATCAG |
| KIF4A | NM_012310.3 | 3232-3331 | GGAGCAAAGCATGGACATCGAGGATCTAAAATATTGTTCAGAGCATTCTGTG AATGAGCATGAGGATGGTGATGGTGATGATGATGAGGGGGATGACGAG |
| MAD2L1 | NM_002358.3 | 183-282 | TGGCCGAGTTCTTCTCATTCGGCATCAACAGCATTTATATCAGCGTGGCATA |
| | | | TATCCATCTGAAACCTTTACTCGAGTGCAGAAATACGGACTCACCTT |
| MCM2 | NM_004526.3 | 1297-1396 | CCATTCTCCTCGCAGATCTGGTGGACAGCTGCAAGCCAGGAGACGAGATAG AGCTGACTGGCATCTATCACAACAACTATGATGGCTCCCTCAACACTGC |
| MCM7 | NM_182776.1 | 1326-1425 | TGGGAAATATCCCTCGTAGTATCACGGTGCTGGTAGAAGGAGAGAACACAA GGATTGCCCAGCCTGGAGACCACGTCAGCGTCACTGGTATTTCTTGCC |
| MELK | NM_014791.2 | 801-900 | TATGAGAGGAAAATATGATGTTCCCAAGTGGCTCTCTCCCAGTAGCATTC TGCTTCTTCAACAAATGCTGCAGGTGGACCCAAAGAAACGGATTTCTATG |
| NCAPH | NM 015341.3 | 1551-1650 | TGCTACTATTCTGACCAAGTCCACTTTGGAGAACCAGAATTGGAGAGCTA CCACCCTTCCTACAGATTTCAACTACAATGTTGACACTCTGGTCCAGCTT |
| NDE1 | NM_001143979.1 | 1245-1344 | AGCGCTTGAATCAGGCCATCGAAAGAAATGCCTTCCTGGAAAGTGAACTT GATGAAAAAGAGAATCTCCTGGAATCTGTTCAGAGACTGAAGGATGAAGC |
| NEK2 | NM_002497.3 | 481-580 | AGAGTTTGTTCTTCGAGTGATGACTCAGTTGACTCTGGCCCTGAAGGAAT GCCACAGACGAAGTGATGGTGGTCATACCGTATTGCATCGGGATCTGAAA |
| NUF2 | NM_031423.3 | 931-1030 | AGATACGGTCCAGAAGCTTAAAAATGCCAGACAAGAAGTGGTGGAGAAAT ATGAAATCTATGGAGACTCAGTTGACTGCCTGCCTTCATGTCAGTTGGAA |
| OIP5 | NM_007280.1 | 669-768 | AGTGCTAACGCACAATCGCTTAAAATCACTAATGAAGATTCTGAGTGAAGT GACTCCTGACCAGTCCAAGCCAGAAAACTGATCCTGTACCAAAGCTTGA |
| PBK | NM_ 018492.2 | 1588-1687 | GCTGATGTGTTTATCAAATGATAACTGGAAGCTGAGGAGAATATGCCTCA AAAAGAGTAGCTCCTTGGATACTTCAGACTCTGGTTACAGATTGTCTTGA |
| PGK1 | NM_000291.2 | 1031-1130 | GCAAGAAGTATGCTGAGGCTGTCACTCGGGCTAAGCAGATTGTGTGGAA TGGTCCTGTGGGGGTATTTGAATGGGAAGCTTTTGCCCGGGGAACCAAAGC |
| PLK4 | NM_014264.4 | 2970-3069 | CTACACAGTTAACTAGTGGAGCTGTGTGGGTTCAGTTTAATGATGGGTCCC AGTTGGTTGTGCAGGCAGGAGTGTCTTCTATCAGTTATACCTCACCAAA |
| PPIA | NM_021130.3 | 201-300 | AGCACTGGAGAGAAAGGATTTGGTTATAAGGGTTCCTGCTTTCACAGAAT TATTCCAGGGTTTATGTGTCAGGGTGGTGACTTCACACGCCATAATGGCA |
| PRC1 | NM_199413.1 | 1136-1235 | ACAGAAAGTCTGCTCCAGCTCCACGATGCTGAGATTGTGCGGTTAAAAAA CTACTATGAAGTTCACAAGGAACTCTTTGAAGGTGTCCAGAAGTGGGAAG |
| PTTG1 | NM_004219.2 | 543-642 | TGTGAAGATGCCCTCTCCACCATGGGAATCCAATCTGTTGCAGTCTCCTT CAAGCATTCTGTCGACCCTGGATGTTGAATTGCCACCTGTTTGCTGTGAC |
| RAD51AP1 | NM_ 001130862.1 | 1126-1225 | TCTGTGAAGTCTCCCAATCAGAGTCTCCGCCTTGGCTTGTCCAGATTAG CACGAGTTAAACCTTTGCATCCAAATGCCACTAGCACCTGAGTGTGGTACA |
| RNASEH2A | NM_006397.2 | 234-333 | AGATCTGGAGGCGCTGAAAGTGGCAGACTCAAAGACCCTATTGGAGAGCG AGCGGGAAAGGCTGTTTGCGAAAATGGAGGACACGGACTTTGTCGGCTGG |
| RPLPO | NM_ 001002.3 | 251-350 | CGAAATGTTTCATTGTGGGAGCAGACAATGTGGGCTCCAAGCAGATGCAG CAGATCCGCATGTCCCTTCGCGGGAAGGCTGTGGTGCTGATGGGCAAGAA |
| RRM2 | NM_ 001034.1 | 1616-1715 | AGGGGTGACCCTTTAGTGAGCTTAGCACAGCGGGATTAAACAGTCCTTTA ACCAGCACAGCCAGTTAAAAGATGCAGCCTCACTGCTTCAACGCAGATTT |
| SDHA | NM_004168.1 | 231-330 | TGGAGGGGCAGGCTTGCGAGCTGCATTTGGCCTTTCTGAGGCAGGGTTT |
| | | | AATACAGCATGTGTTACCAAGCTGTTTCCTACCAGGTCACACACTGTTGCA |
| SGO2 | NM_152524.5 | 3361-3460 | AGACCTCCATAGATCCTTCTCCAGAGAGCCATGAAGTAATGGAAAGAATA CTTGACAGCGTTCAGGGAAAGTCTACTGTATCTGAACAAGCTGATAAGGA |
| SMC2 | NM_001042551.1 | 1856-1955 | AAGGACTTGTGGCTTCTCTGATTAGTGTGAAAGACACTTCTGCAACCACA GCTTTAGAATTAGTGGCTGGAGAACGACTCTACAATGTTGTAGTAGACAC |
| SPAG5 | NM_006461.3 | 511-610 | AACAAGACATGATATTTGAGGCCCGTTTAGATACCATGGCAGAGACAAAC AGCATATCTTTAAATGGACCTTTGAGAACAGACGATCTGGTGAGAGAGGA |
| SPC25 | NM_020675.3 | 327-426 | AATCAGATCAGCAGGCAAAATAAGCTCATTCAAGAAAAAAAGGATAACTT GTTAAAATTGATTGCTGAAGTAAAAGGCAAAAAGCAGGAATTGGAAGTAC |
| TOP2A | NM_ 001067.3 | 3564-3663 | TTTAACCAAGGAAAAGAAAGATGAACTCTGCAGGCTAAGAAATGAAAAAG AACAAGAGCTGGACACATTAAAAAGAAAGAGTCCATCAGATTTGTGGAAA |
| TPX2 | NM_012112.4 | 2976-3075 | TGGGACCTGCTCTTAACCTCAAACCTAGGACCGTCTTGCTTTGTCATTGG GCATGGAGAGAACCCATTTCTCCAGACTTTTACCTACCCGTGCCTGAGAA |
| TRIP 13 | NM_004237.2 | 451-550 | AAGAGACAGAAAACATAATTGCAGCAAATCACTGGGTTCTACCTGCAGCT GAATTCCATGGGCTTTGGGACAGCTTGGTATACGATGTGGAAGTCAAATC |
| TTK | NM_003318.3 | 1201-1300 | AACCAGAGGTTCCAGAGAGTAACCAGAAACAGTGGCAATCTAAGAGAAAG TCAGAGTGTATTAACCAGAATCCTGCTGCATCTTCAAATCACTGGCAGAT |
| UBE2C | NM_007019.2 | 562-661 | CTTTTAAGAAGTACCTGCAAGAAACCTACTCAAAGCAGGTCACCAGCCAG GAGCCCTGACCCAGGCTGCCCAGCCTGTCCTTGTGTCGTCTTTTTAATTT |
| ZWINT | NM_007057.3 | 951-1050 | AAAGACTGTGAACTCCTGAGTTCAGCTTGATTTCTGACTACATCCCAGCA AGCTCTGGCATCTGTGGATTAAAATCCCTGGATCTCTCTCAGTTGTGTAT |

### Statistical analysis

NanoString data were normalized using the manufacturer's software (nSolver v3.0) and recommendations. Background subtraction was performed on negative control probes and normalization with positive control probes and housekeeping genes was performed with the geometric mean method. Both RNA-seq and nanoString expression values were log2-transformed. As previously described, we used the nearest centroid method to determine CINSARC risk groups. To apply the signature independently on each sample, we performed leave-one-out cross-validation by computing distances from a sample to centroids (good and poor prognoses) of all other samples using the same technique. Metastasis-free survival was analyzed by the Kaplan-Meier estimator from the original diagnosis to diagnosis of the metastatic event or the last follow-up. Significance was expressed by the Cox proportional hazards regression model implemented in the survival package (v2.41-3) available in R (v3.4.2).

### RESULTS

### Cohorts

Tumor characteristics and follow-up of patients in both cohorts are presented in Table 5.

**Table 5: Characteristics of processed cohorts**

| Cohort | #1 (n=124) | #2 (n=47) |
|---|---|---|
| Expression quantification | RNA-seq and NanoString | RNA-seq and NanoString |
| Material | Fresh-frozen tissue | R: 47 (FFT); 41 (FFPE) |
| | | N: 47 (FFT); 47 (FFPE) |
| Median age at diagnosis (years) [95% CI] | 63 [60-65] | 63 [60-66] |
| Histotype (%) | | |
| Dedifferentiated liposarcoma | 15 (12.10) | 6 (12.77) |
| Gastrointestinal stromal | 12 (9.68) | 11 (23.40) |
| tumor | | |
| Leiomyosarcoma | 32 (25.81) | 15 (31.91) |
| Myxofibrosarcoma | 11 (8.87) | 4 (8.51) |
| Undifferentiated pleomorphic sarcoma | 39 (31.45) | 10 (21.28) |
| Others | 15 (12.10) | 1 (2.13) |
| Gender (%) | | |
| Female | 61 (49.19) | 29 (61.7) |
| Male | 63 (50.81) | 18 (38.3) |
| FNCLCC grade (%) | | |
| land 2 | 32 (25.81) | 9 (19.15) |
| 3 | 74 (59.68) | 25 (53.19) |
| Unknown | 6 (4.84) | 2 (4.26) |
| NA (GIST) | 12 (9.68) | 11 (23.40) |
| Surgical margin (%) | | |
| R0 | 53 (42.74) | 29 (61.70) |
| R1 | 42 (33.87) | 15 (31.91) |
| R2 | 3 (2.42) | 0 |
| Unknown or NA | 26 (20.97) | 3 (6.38) |
| Median follow-up (month) [95% CI] | 57.31 [45.27-66.60] | 57.33 [47.01-67.58] |
| Local recurrence (%) | 39 (31.45) | 12 (25.53) |
| Metastasis (%) | 50 (40.32) | 13 (27.66 |

Others cohort #1: two low-grade fibromyxoid sarcomas, two malignant solitary fibrous tumors, six pleomorphic liposarcomas, four pleomorphic rhabdomyosarcomas and one synovial sarcoma. Others cohort #2: one pleomorphic liposarcoma; FFT: fresh-frozen tissue; FFPE: formalin-fixed, paraffin-embedded; R: RNA sequencing; N: nanoString.

### NanoString nCounter® gene expression system performance

First the reproducibility of the nCounter® (nanoString Technologies) system in measuring the NanoCind panel was examined. The raw counts for all 75 genes on the same sample from two independent hybridizations of RNA from fresh-frozen sarcoma samples are shown Figure 3A (between two independent cartridges) and 3B (in the same cartridge). A linear fit to the data resulted in a median Pearson's correlation coefficient of 0.9849 (inter- and intra-cartridges). Then, we examined the linearity and dynamic range of two fresh-frozen sarcoma samples. Figure 3 shows the results for all 75 genes from each hybridization reaction with inputs of 100ng (reference input), 300ng, 600ng and 1000ng of RNA from the same fresh-frozen sarcoma samples. Raw points demonstrated a linear correlation between different inputs of the same sample (Figure 3C). The histograms (Figure 3D) were established on normalized counts by the positive controls and made it possible to evaluate the average signal increase factor induced by the increase in inputs. Median fold changes (3, 6.1 and 10.2) are very close to the expected multiplicative factors (3, 6 and 10).

### Technological transfer: from RNA-seq to nanoString (NanoCind)

We performed comparisons of the same fresh-frozen samples (cohort #1, n=124) between both techniques. Pearson's correlation of 67 CINSARC gene expression values for the same high quality RNA samples between RNA-seq and nanoString was 0.797±0.130 (Figure 4B). Thus, we aimed to test the prognostic value of CINSARC. An agreement test between the prognostic groups (CINSARC C1, low-risk and CINSARC C2, high-risk) by nanoString on fresh-frozen samples resulted in a similar CINSARC classification for 107 out of 124 transcriptomic profiles (86% agreement; Cohen's kappa=0.687; P<10-10) compared to RNA-seq classification on fresh-frozen samples. By combining associated clinical data outcome, we performed metastasis-free survival analysis. NanoString had a significant prognostic value (P = 1.01 x 10 -2, HR=2.9, 95% CI: 1.23-6.82) (Figure 1A) while RNA-seq did not (P = 9.68 x 10 -2, HR=1.69, 95% CI: 0.89-3.2)(Figure 1B, Table 6).

**Table 6: Prognostic analyses for metastasis-free survival according to CINSARC signature and to FNCLCC grading system according to cohorts, materials and technologies**

| **Characteristics** | ***n*** | p-value | **HR [95%CI]** |
|---|---|---|---|
| **Cohort #1** | | | |
| CINSARC, RNA-seq, fresh-frozen tissue (C1 vs C2) | 124 | 0.0968 | 1.69 [0.89-3.2] |
| CINSARC, NanoString (NanoCind), fresh-frozen tissue (C1 vs C2) | 124 | **0.0101** | 2.9 [1.23-6.82] |
| **Cohort #2** | | | |
| CINSARC, RNA-seq, FFPE (C1 vs C2) | 17 | 0.134 | 5.12 [1.61-5.49] |
| CINSARC, NanoString (NanoCind), FFPE (C1 vs C2) | 47 | **0.0421** | 3.53 [0.96-13.06] |
| **Pooled cohorts #1 and #2** | | | |
| FNCLCC grade (G1/G2 vs G3) | 116 | 0.658 | 1.16 [0.6-2.26] |
| | 116 | 0.116 | 1.74 [0.88-3.53] |
| CINSARC, RNA-seq (C1 vs C2) | 116 | **0.00939** | 3.58 [1.28-10] |
| CINSARC, NanoString (NanoCind) (C1 vs C2) | | | |

FNCLCC, Fédération Nationale des Centres de Lutte Contre le Cancer; G1, grade 1 FNCLCC; G2, grade 2 FNCLCC; G3, grade 3 FNCLCC; CINSARC, Complexity INdex in SARComas; C1, low-risk CINSARC; C2, high-risk CINSARC; HR, hazard ratio; CI, confidence interval; FFPE, formalin-fixed, paraffin-embedded. Significant P-value < 0.05 are in bold.

### Material transfer: from fresh-frozen tissue to FFPE blocks

To optimize the quantity and quality of RNA extraction, we performed different extractions on the same FFPE blocks with different commercial kits. Mean Pearson's correlation of the raw counts with the NanoCind panel for the High Pure FFPET RNA Isolation Kit, Roche (reference kit) compared to the Roche FFPET RNA Isolation Kit (used in Prosigna)(n=2) was 0.993 (Figure 5A and 5B). Mean Pearson's correlation of the raw counts with the NanoCind panel for the High Pure FFPET RNA Isolation Kit, Roche (reference kit) compared to the RNeasy FFPE kit, Qiagen Kit (n=27) was 0.786 (range, 0.104-0.984) (Figure 5C). Moreover, we assessed the stability of RNA from unstained slides following extraction at different times after cutting with a view to using this technique in routine practice. On the same FFPE block, we performed RNA extractions on unstained slides the same day of the cut (J0, reference protocol) and 21 days room temperature storage after the day of cutting (J21). Pearson's correlation for interval extraction replicate was 0.990 (Figure 5D).

Thus, we performed comparisons of paired fresh-frozen and FFPE samples (cohort #2, n=47) with nanoString. NanoString provided a good correlation between all 47 pairs of fresh-frozen and FFPE RNA (mean Pearson's r = 0.651±0.1178; Figure 4A). Moreover, Pearson's correlation of 67 CINSARC gene expression values for the same samples (cohort #2) between RNA-seq on fresh-frozen samples (reference material and technique) and nanoString on FFPE blocks was 0.687±0.104 (Supplementary Figure 2C).
Then, we tested the prognostic value of the CINSARC signature evaluated by nanoString on FFPE samples. The agreement test between prognostic groups (CINSARC C1, low-risk and CINSARC C2, high-risk) by nanoString on FFPE samples resulted in a similar CINSARC classification for 38 out of 45 transcriptomic profiles (84% agreement; Cohen's kappa=0.688; P=3.79 x10-6) compared to RNA-seq classification on fresh-frozen samples (reference technique and materials). Moreover, nanoString had a significant prognostic value (P = 4.21 x 10 -2, HR=3.53, 95% CI: 0.96-13.06) (Figure 1C, Table 3) on FFPE samples.

Finally, we performed metastasis-free survival analysis of the pooled fresh-frozen and FFPE samples (n=141) with RNA-seq and nanoString (Figure 1D). Both techniques provided a significant prognostic value but the CINSARC signature with nanoString (NanoCind) (P = 2.45 x 10 -3, HR=3.19, 95% CI: 1.43-7.08) was more discriminating than that obtained by RNA sequencing (P = 3.42 x 10 -2, HR=1.94, 95% CI: 1.03-3.64). We also investigated the performance of CINSARC in subjects of the same histological grade. Among the pooled fresh-frozen and FFPE samples (n=141) with data obtained by RNA-seq and nanoString, the FNCLCC histological grade was available for 116 cases. The CINSARC signature with nanoString (NanoCind) discriminated two groups of low- and high-risk subjects with clearly significant and different outcomes (P = 9.39 x 10 -3, HR=3.58, 95% CI: 1.28-10, Figure 2A), whereas its prognostic value with RNA-seq was not significant (P = 1.16 x 10 -1, HR=1.74, 95% CI: 0.86-3.53, Figure 2B) and histological grade (G1/G2 versus G3) did not predict outcome (P = 6.58 x 10 - 1, HR=1.16, 95% CI: 0.6-2.26, Figure 2C). Likewise, in grade 2 tumors, the CINSARC signature (NanoCind) identified two groups of different outcomes with a significant prognostic value (P = 5.1 x 10 -3, HR=10.95, 95% CI: 1.38-86.93, Figure 2D).

### DISCUSSION

Soft-tissue sarcomas are a group of rare, heterogeneous, aggressive tumors with high metastatic risk. The CINSARC signature outperforms histological grade for metastatic prognosis in several cancer types and especially in soft tissue sarcomas (Chibon *et al.* **([5]);** Lagarde *et al.* **([6]);** Lagarde *et al.* **([7])).** Nevertheless, it is not applicable in routine clinical practice because it was developed and validated in microarrays and RNA sequencing, which are techniques requiring large amounts of high-quality RNA, especially from fresh-frozen tumor tissue. In the current study, the CINSARC signature established by nanoString (NanoCind) on FFPE blocks was a strong predictor for metastatic events (which outperformed histological grade) and is therefore usable in routine clinical settings and clinical trials.

In the first part of the CINSARC transfer, CINSARC gene expression values correlated well using these two techniques (mean Pearson's r is 0.797 between RNA-seq and nanoString), which is consistent with already published methodological correlations (Geiss *et al.* **([10]);** Malkov *et al.* (**[11]**); Northcott *et al.* **([12]);** Reis *et al.* **([13])).** Furthermore, similar risk-group classifications (86%) were noted. However, while its prognostic value with RNA-seq was non-significant (reference technique, P = 9.68 x 10⁻²), it was significant with nanoString (NanoCind) (eP = 1.01 x 10⁻²).

In recent decades, high-throughput molecular analyses, especially gene expression profiling, have provided insights into the extensive heterogeneity of cancers and have led to the identification of specific gene expression signatures as a prognostic factor in several malignancies such as breast cancer, lymphoma and hepatocellular carcinoma (Perou, C. M. et al. Molecular portraits of human breast tumours. Nature 406, 747-752 (2000) **([15]);** Lossos, I. S. et al. Prediction of survival in diffuse large-B-cell lymphoma based on the expression of six genes. N. Engl. J. Med. 350,1828-1837 (2004) **([16]);** Hoshida, Y. et al. Gene expression in fixed tissues and outcome in hepatocellular carcinoma. N. Engl. J. Med. 359, 1995-2004 (2008) **([17])).** For example, several multigene signatures such as Oncotype™ or Prosigna™ are marketed for early-stage breast cancer and are used in routine practice for therapeutic management. No similar signature is currently available in routine practice for sarcoma patients. Indeed, although Chibon *et al.* identified and validated a prognostic molecular signature in sarcomas (CINSARC) in 2010, they worked on frozen tumor material that was evaluated by microarrays (**[5]**), a technique that is not widely available. In 2016, Lesluyes *et al.* successfully transferred the signature using RNA sequencing with frozen tissues. However, their attempt to adapt it on FFPE blocks was disappointing with more than 50% of FFPE cases that could not be analyzed with RNA-seq owing to degradation of RNA induced by formalin **([8]).** In our study, all (n=47) FFPE blocks (the same cases as those tested by Lesluyes *et al.*) were analyzable with nanoString. Moreover, in terms of prognosis, the CINSARC signature obtained with nanoString (NanoCind) from RNA extracted from FFPE blocks had a significant prognostic value (P = 4.21 x 10 -2, HR=3.53, 95% CI: 0.96-13.06), allowing its use in routine practice. The low quality of RNA extracted from FFPE blocks did not influence the efficiency of the results when nanoString was used. These findings are in accordance with previous studies reporting the robust performance of nanoString on RNA extracted from FFPE material with results comparable to those obtained with RNA from fresh-frozen tissue by microarrays **([14]).** Indeed, nanoString relies on relatively short pairs of 50mer probes and does not require reverse transcription of mRNA and subsequent cDNA amplification. This feature offers advantages over NGS and PCR-based methods, including the absence of amplification bias, which may be higher when using fragmented RNA isolated from FFPE specimens. The value and the superiority of nanoString (NanoCind) were confirmed by three of our results: first, it gave a significant prognostic value which was more discriminating than that established by RNA sequencing (P = 2.45 x 10⁻³ vs. P = 3.42 x 10⁻²); second, it outperformed the current histological grade (FNCLCC) (P = 9.39 x 10⁻³ vs. P = 6.58 x 10⁻¹); and third, in grade 2 (FNCLCC) tumors, it clearly and significantly differentiated two groups of different outcomes (P = 5.1 x 10⁻³). Its advantage over histologic grading is that it stratifies patients into two groups instead of three, which is essential for facilitating clinical management.

To be able to use this signature in routine clinical practice, we tested it in several conditions. It showed excellent reproducibility (replicates averaging R2 of 0.983) and robustness (good linearity of quantification), consistent with previous reports **([10]).** Moreover, we tested three different commercial nucleic acid extraction kits for FFPE material. The two we tested demonstrated good results with high (Pearson's r = 0.786 for RNeasy FFPE kit, Qiagen) to excellent (Pearson's r = 0.993 for Roche FFPET RNA Isolation Kit, used in Prosigna) correlation. Finally, the test turnaround time with nanoString is shorter than for NGS approaches; library preparation and nucleic acid amplification are not required and the complexity of data analysis is negligible compared with NGS, which requires a specialized bioinformatics pipeline and sequence analysis strategies.

In conclusion, nanoString could be used for accurate gene expression quantification and to determine the CINSARC signature using FFPE sarcoma samples in routine practice. The CINSARC signature with nanoString (NanoCind) outperformed the current histological FNCLCC grading system and predicted metastatic clinical outcome, which is useful for personalized therapeutic management. Moreover, Lesluyes *et al.,* recently reported that the CINSARC can be considered as an excellent prognostic marker for clinical outcome in many other human malignancies (significant survival differences between risk groups in 33 published studies representing 17 tumor types) **([14]).**

### Reference List

1- Fletcher CDM, Bridge JA, Hogendoorn PCW, et al. World Health Organization classification of tumours: Pathology and genetics of tumours of soft tissue and bone. Lyon, France: IARC Press 2012.
2- Trojani M, Contesso G, Coindre JM et al. Soft-tissue sarcomas of adults; study of pathological prognostic variables and definition of a histopathological grading system. Int J Cancer 1984;33:37e42.
3- Italiano A, Delva F, Mathoulin-Pelissier S et al. Effect of adjuvant chemotherapy on survival in FNCLCC grade 3 soft tissue sarcomas: a multivariate analysis of the French Sarcoma Group Database. Ann Oncol 2010; 21: 2436-2441.
4- Coindre JM, Terrier P, Guillou L et al. Predictive value of grade for metastasis development in the main histologic types of adult soft tissue sarcomas: a study of 1240 patients from the French Federation of Cancer Centers Sarcoma Group. Cancer 2001;91:1914e26.
5- Chibon F, Lagarde P, Salas S et al. Validated prediction of clinical outcome in sarcomas and multiple types of cancer on the basis of a gene expression signature related to genome complexity. Nat Med. 2010; 16:781-7.
6- Lagarde P, Pérot G, Kauffmann A, et al. Mitotic checkpoints and chromosome instability are strong predictors of clinical outcome in gastrointestinal stromal tumors. Clin Cancer Res 2012;18:826e38.
7- Lagarde P, Przybyl J, Brulard C, et al. Chromosome instability accounts for reverse metastatic outcomes of pediatric and adult synovial sarcomas. J Clin Oncol 2013;31 :608e15.
8- Lesluyes T, Pérot G, Largeau MR, et al. RNA sequencing validation of the Complexity INdex in SARComas prognostic signature. Eur J Cancer 2016; 22:104-111.
9- Von Ahlfen S, Missel A, Bendrat K, Schlumpberger M: Determinants of RNA quality from FFPE samples. PLoS One 2007, 2(12):e1261.
10-Geiss GK, Bumgarner RE, Birditt B, Dahl T, Dowidar N, et al. (2008) Direct multiplexed measurement of gene expression with color-coded probe pairs. Nat Biotechnol 26(3): 317-325.
11-Malkov VA, Serikawa KA, Balantac N, Watters J, Geiss G, et al. (2009). Multiplexed measurements of gene signatures in different analytes using the Nanostring nCounter Assay System. BMC Res Notes 2: 80.
12-Northcott PA, Shih DJ, Remke M, Cho YJ, Kool M, et al. (2012) Rapid, reliable, and reproducible molecular sub-grouping of clinical medulloblastoma samples. Acta Neuropathol 123(4): 615-626.
13-Reis PP, Waldron L, Goswami RS, Xu W, Xuan Y, Perez-Ordonez B, Gullane P, Irish J, Jurisica I, Kamel-Reid S (2011) mRNA transcript quantification in archival samples using multiplexed, color-coded probes. BMC Biotechnol 11:46.
14-Lesluyes T, Delespaul L, Coindre JM, Chibon F. The CINSARC signature as a prognostic marker for clinical outcome in multiple neoplasms. Sci Rep. 2017; 14;7(1):5480.
15-Perou, C. M. et al. Molecular portraits of human breast tumours. Nature 406, 747-752 (2000).
16-Lossos, I. S. et al. Prediction of survival in diffuse large-B-cell lymphoma based on the expression of six genes. N. Engl. J. Med. 350,1828-1837 (2004).
17-Hoshida, Y. et al. Gene expression in fixed tissues and outcome in hepatocellular carcinoma. N. Engl. J. Med. 359, 1995-2004 (2008).
18- WO2010/019826.
19. WO02010/122243.

## Claims

1. Method for determining *in vitro* the presence and/or the level of target molecules in a biological sample, comprising the steps of:
(1) placing said biological sample in the presence of a pool of different probes, each probe comprising (a) a binding region specific of one target molecule, and eventually (b) a detectable region that is unique for each of the at least one target molecule, wherein the binding region of a probe comprises a polynucleotide having a sequence selected in the group consisting of SEQ ID NO: 1 to SEQ ID NO: 67 or of SEQ ID NO: 1 to SEQ ID NO: 75, and wherein the presence of a target molecule in the biological sample allows the formation of a specific detectable molecular complexes between said probe and said target molecule, and
(2) detecting and/or counting the molecular complex(es) formed, thus allowing the determination of the presence and/or of the level of said at least one target molecule in the biological sample.

2. Method according to claim 1, wherein said biological sample is a fresh or a frozen sample or a formalin-fixed, paraffin-embedded (FFPE) block.

3. Method according to anyone of the preceding claims, wherein step (2) comprises an individual counting of the detectable region of the probe.

4. Method according to claim 1, wherein said detectable region comprises a detectable signal chosen among fluorescence, luminescence, colorimetry and a magnetic field.

5. Method according to claim 1, wherein said detectable region comprises fluorochromes defining a unique detectable signature.

6. Use of a method as defined in anyone of the preceding claims, for the *in vitro* analysis of a cancer.

7. Use according to claim 6, wherein said cancer is chosen among soft-tissue sarcomas (STS) and gastrointestinal stromal tumors (GIST).

8. Use according to claim 7, for predicting, in said cancer, the occurrence of metastases, or for distinguishing subgroups of patients of good or bad prognosis.

9. Pool of polynucleotides comprising the polynucleotides of sequence SEQ ID NO : 1 to SEQ ID NO : 67.

10. Pool of polynucleotides according to claim 9, comprising in addition the polynucleotides of sequence SEQ ID NO : 68 to SEQ ID NO : 75.

11. Pool of polynucleotides according to claim 9, consisting of polynucleotides of sequence SEQ ID NO : 1 to SEQ ID NO : 67.

12. Pool of polynucleotides according to claim 10, consisting of polynucleotides of sequence SEQ ID NO : 1 to SEQ ID NO : 75.

13. Use of a pool of polynucleotides as defined in anyone of claims 9 to 12, in a method for determining *in vitro* the presence and/or the level of at least one target molecule in a biological sample.

14. Kit comprising :
- a pool of probe as defined in claim 1, capable of specifically forming a molecular complex with a target molecule, and
- means for detecting and/or quantifying said molecular complex.

15. Use of a kit as defined in claim 14, for *in vitro* predicting the occurrence of metastases, and/or for distinguishing subgroups of good or bad prognosis in patients having a cancer, such as soft-tissue sarcomas (STS) or gastrointestinal stromal tumors (GIST).


SEQUENCE LISTING

<110>  UNIVERSITÉ DE BORDEAUX
       INSTITUT NATIONAL DE LA SANTÉ ET DE LA RECHERCHE MÉDICALE -
        INSERM
       INSTITUT BERGONIÉ
       UNIVERSITÉ PAUL SABATIER TOULOUSE III
       INSTITUT CLAUDIUS REGAUD

<120>  Method for determining the in vitro presence of a target molecule
        in a biological sample, and its use for the in vitro analysis of
        cancers, in particular soft-tissue sarcomas  and
       gastrointestinal stromal tumors

<130>  BNT223855EP00

<160>  75

<170>  PatentIn version 3.5

<210>  1
<211>  100
<212>  DNA
<213>  Homo sapiens

<400>  1
atgacctctt cagtgatgtc ctagaggaag gtgaactaga tatggagaag agccaagagg      60

agatggatca agcattagca gaaagcagcg aagaacagga                            100


<210>  2
<211>  100
<212>  DNA
<213>  Homo sapiens

<400>  2
tgatccaagc acattataga ggatataaag gaaggcaggt ctttcttcgg cagaaatctg      60

ctgctttgat catacaaaaa tatatacgag ccagggaggc                             100


<210>  3
<211>  100
<212>  DNA
<213>  Homo sapiens

<400>  3
agctccagtt ggaggtccaa aacgtgttct cgtgactcag caatttcctt gtcagaatcc      60

attacctgta aatagtggcc aggctcagcg ggtcttgtgt                            100


<210>  4
<211>  100
<212>  DNA
<213>  Homo sapiens

<400>  4
agatgctcta atgtactgcc atgggaagaa ggtgattcac agagacataa agccagaaaa      60

tctgctctta gggctcaagg gagagctgaa gattgctgac                            100

<210>  5
<211>  100
<212>  DNA
<213>  Homo sapiens

<400>  5
ccattctaag tcattgggga aacggggtga acttcaggtg gatgaggaga cagaatagag      60

tgataggaag cgtctggcag atactccttt tgccactgct                            100


<210>  6
<211>  100
<212>  DNA
<213>  Homo sapiens

<400>  6
atttgcagat caatctcctg gaaacctcag ttcttcatcc ctcagaagaa agctgttttt      60

agatgggaac ggaagcatct ccgactcctt accttcggct                            100


<210>  7
<211>  100
<212>  DNA
<213>  Homo sapiens

<400>  7
gaagataaag aaaatgtggt agcaaaacag tgtacccagg cgactttgga ttcttgtgag      60

gaaaacatgg tggtgccttc aagggatgga aaattcagtc                            100


<210>  8
<211>  100
<212>  DNA
<213>  Homo sapiens

<400>  8
gagtcttctg taccacaacg aagcacacta gctgaactaa agagcaaagg gaaaaagaca      60

gcaagagctc caatcatccg tgtaggaggt gctctcaagg                            100


<210>  9
<211>  100
<212>  DNA
<213>  Homo sapiens

<400>  9
cgggacaaag ctggcctgaa tcattaatac gaaagactgg atataccctg gaaagtctta      60

agccttgtct catggacctt caccagacct acctcaaagc                            100


<210>  10
<211>  100
<212>  DNA
<213>  Homo sapiens

<400>  10
aacttgagga agagcaagca gtcagaccaa aatacctact gggtcgggaa gtcactggaa      60

acatgagagc catcctaatt gactggctag tacaggttca                          100

<210>  11
<211>  100
<212>  DNA
<213>  Homo sapiens

<400>  11
aggttgatgt tgaacagcac actttagcca agtatttgat ggagctgact ctcatcgact      60

atgatatggt gcattatcat ccttctaagg tagcagcagc                          100

<210>  12
<211>  100
<212>  DNA
<213>  Homo sapiens

<400>  12
ggaacatcag aaagcctggg ctttgaacct gaacggtttt gatgtagagg aagccaagat      60

ccttcggctc agtggaaaac cacaaaatgc gccagagggt                          100

<210>  13
<211>  100
<212>  DNA
<213>  Homo sapiens

<400>  13
agctcccgga tgctgcacaa ccattttgac ctctcagtaa ttgagctgaa agctgaggat      60

cggagcaagt ttctggacgc acttatttcc ctcctgtcct                          100

<210>  14
<211>  100
<212>  DNA
<213>  Homo sapiens

<400>  14
ggggaagtta tatgaagcct acagtaaagt ctgtcgcaaa cagcaggtgg cggctgtgga      60

ccagtcagag tgtttgtcac tttcagggct cttggaagcc                          100

<210>  15
<211>  100
<212>  DNA
<213>  Homo sapiens

<400>  15
atcccacata atcacaggaa acaagattcc actgagtggc ccagtaccta aggagctgga      60

tcagcagtcc accacaaaag cttctgttaa aagaccctac                          100

<210>  16
<211>  100
<212>  DNA
<213>  Homo sapiens

<400>  16
agacttcagt ggtctcagtt ccctgctgct tgagcagtca cctgttcctg agccattacc      60

tcaaccagat tttgatgaca agggggagaa tcttgaaaac                            100


<210>  17
<211>  100
<212>  DNA
<213>  Homo sapiens

<400>  17
gcctaccagc aggggagcaa ctggagggtc ttaaacatgc ccaggactca gatccccgct      60

ctcctactct tggtattgca cggacaccta tgaagaccag                            100


<210>  18
<211>  100
<212>  DNA
<213>  Homo sapiens

<400>  18
tccctgttta ctgaagacca aatactggtt tggagacaac ttccatgtct tgctcttcta      60

cctccctagt tagtggaaat ttggataagg gaactgtagg                            100


<210>  19
<211>  100
<212>  DNA
<213>  Homo sapiens

<400>  19
ggtacctatg gagttgtgta taagggtaga cacaaaacta caggtcaagt ggtagccatg      60

aaaaaaatca gactagaaag tgaagaggaa ggggttccta                            100


<210>  20
<211>  100
<212>  DNA
<213>  Homo sapiens

<400>  20
cactttgagc agttgcctgg aaggctgggc atttccatca tatagacctc tgcccttcag      60

agtagcctca ccattagtgg cagcatcatg taactgagtg                            100


<210>  21
<211>  100
<212>  DNA
<213>  Homo sapiens

<400>  21
gcaccaatca tcgattctgc catacaaggc tacaatggta ctatatttgc ctatggacag      60

actgcttcag gaaaaacata taccatgatg ggttcagaag                            100


<210>  22

<211>  100
<212>  DNA
<213>  Homo sapiens

<400>  22
gtggctacta ctgaatttct ttggtctgta ccctgtagcc ctcaaagtct ggacatttct        60

ttcgcaatac atccagagga tgcaaaagct ctatgggaca                              100


<210>  23
<211>  100
<212>  DNA
<213>  Homo sapiens

<400>  23
gtactaccgc attgcttgaa cagctggaag agacaacgag agaaggagaa aggagggagc        60

aggtgttgaa agccttatct gaagagaaag acgtattgaa                              100


<210>  24
<211>  100
<212>  DNA
<213>  Homo sapiens

<400>  24
agggtgatgg attggagttc aagagacact tcctgaagat taaagggaag ctgattgata        60

ttgtgagcag ccagaagatt tggcttcctg ccacatgatc                              100


<210>  25
<211>  100
<212>  DNA
<213>  Homo sapiens

<400>  25
ttgctaaagc ccttttgtgc tgcactactt aagcacatca atgattctgc tcctgaagtc        60

agagatgccg catttgaagc attgggtact gctttgaagg                              100


<210>  26
<211>  100
<212>  DNA
<213>  Homo sapiens

<400>  26
aaactcatct gatgtctgaa gaggagtgga ggagacttgg tgtccaacag agtctaggct        60

gggttcatta catgattcat gagccagaac cacatattct                              100


<210>  27
<211>  100
<212>  DNA
<213>  Homo sapiens

<400>  27
cagttatttc aagtaccatt ggaagaggaa ggacaacgtg gtggacctat ccttgcacca        60

gaggagatta agactatttt tggtagcatc ccagatatct                              100

<210>  28
<211>  100
<212>  DNA
<213>  Homo sapiens

<400>  28
cagggacttc acctctacac tgtggtggtt tatgactttg cccaaggctg tcagatagtt        60

gatttggctg acctgaccca actagtggac agttgtaaat                              100


<210>  29
<211>  100
<212>  DNA
<213>  Homo sapiens

<400>  29
tgagtgggga agaatgtaag aaacagttga ttaacaccct gtgttctggc aggtgggatc        60

agcaatatgt aatccaactc acctccatgt tcaaggatgt                              100


<210>  30
<211>  100
<212>  DNA
<213>  Homo sapiens

<400>  30
gcacaactca gtgacatgga cttaatcaat gtgtctaaag tgagcacaac ttggaagaag        60

atcctagaag atgataaggg ggcattccag ttgtacagta                              100


<210>  31
<211>  100
<212>  DNA
<213>  Homo sapiens

<400>  31
caattcgcca tcaacagcac tgagaggaag cgcatgactt tgaaagacat ctatacgtgg        60

attgaggacc actttcccta ctttaagcac attgccaagc                              100


<210>  32
<211>  100
<212>  DNA
<213>  Homo sapiens

<400>  32
ccgccccatt tcccttccag caaactcaac tcggcaatcc aagcacctag ataccagcac        60

aagtcggtta atccctgtct ggactgagcc tccgttggct                              100


<210>  33
<211>  100
<212>  DNA
<213>  Homo sapiens

<400>  33

agcttgctga aggggaggaa gaaaagccag aaccagacat aagttcagag gaatctgtct 60

ccactgtaga agaacaagag aatgaaactc cacctgctac 100

<210> 34
<211> 100
<212> DNA
<213> Homo sapiens

<400> 34
cagaccattt aatttggcag agcggaaagc tagcgcccat tcaatagtag aatgtgatcc 60

tgtacgaaaa gaagttagtg tacgaactgg aggattggct 100

<210> 35
<211> 100
<212> DNA
<213> Homo sapiens

<400> 35
tgatcgagca atccagtcac ttactattca gactgcatgt gcttttgagc agctagtagt 60

gctaatgaaa cactggctga gtgatttact gccttgtacc 100

<210> 36
<211> 100
<212> DNA
<213> Homo sapiens

<400> 36
aattccgaga ggatcaaata atacgcttgg aaaagctcca caaggaatcc cggggaggtt 60

ttctgcctga ggagcaggat cgtttgctct cagaattaag 100

<210> 37
<211> 100
<212> DNA
<213> Homo sapiens

<400> 37
catagacttg caatgttgaa aactcgtcgc tcctacctgg agaaaaggag ggaggaggaa 60

ttgaagcaat ttgatgagaa tactaattgg ctccatcgtg 100

<210> 38
<211> 100
<212> DNA
<213> Homo sapiens

<400> 38
cacttccttg aagaggagtg tctacatcga aagtcggata ggtaccagca ccagcttcga 60

cagtggcatt gctgggctct cttctatcag tcagtgtacc 100

<210> 39
<211> 100
<212> DNA

<213>  Homo sapiens

<400>  39
tgtgtggatg atttctcgaa agccatgcca gaagcagtct tccaggtcat cttgtagaac      60

tccagctttg ttgaaaatca cggacctcag ctacatcata                          100


<210>  40
<211>  100
<212>  DNA
<213>  Homo sapiens

<400>  40
cctgctctaa cggggcgctg attccaggca atttatccaa ggaagaggag gaactgtctt      60

cccagatgtc cagctttaac gaagccatga ctcagatcag                          100


<210>  41
<211>  100
<212>  DNA
<213>  Homo sapiens

<400>  41
ggagcaaagc atggacatcg aggatctaaa atattgttca gagcattctg tgaatgagca      60

tgaggatggt gatggtgatg atgatgaggg ggatgacgag                          100


<210>  42
<211>  100
<212>  DNA
<213>  Homo sapiens

<400>  42
tggccgagtt cttctcattc ggcatcaaca gcattttata tcagcgtggc atatatccat      60

ctgaaacctt tactcgagtg cagaaatacg gactcacctt                          100


<210>  43
<211>  100
<212>  DNA
<213>  Homo sapiens

<400>  43
ccattctcct cgcagatctg gtggacagct gcaagccagg agacgagata gagctgactg      60

gcatctatca caacaactat gatggctccc tcaacactgc                          100


<210>  44
<211>  100
<212>  DNA
<213>  Homo sapiens

<400>  44
tgggaaatat ccctcgtagt atcacggtgc tggtagaagg agagaacaca aggattgccc      60

agcctggaga ccacgtcagc gtcactggta ttttcttgcc                          100

<210>  45
<211>  100
<212>  DNA
<213>  Homo sapiens

<400>  45
tatgagagga aaatatgatg ttcccaagtg gctctctccc agtagcattc tgcttcttca      60

acaaatgctg caggtggacc caaagaaacg gatttctatg                            100


<210>  46
<211>  100
<212>  DNA
<213>  Homo sapiens

<400>  46
tgctactatt ctgaccaagt ccactttgga gaaccagaat tggagagcta ccacccttcc      60

tacagatttc aactacaatg ttgacactct ggtccagctt                            100


<210>  47
<211>  100
<212>  DNA
<213>  Homo sapiens

<400>  47
agcgcttgaa tcaggccatc gaaagaaatg ccttcctgga aagtgaactt gatgaaaaag      60

agaatctcct ggaatctgtt cagagactga aggatgaagc                            100


<210>  48
<211>  100
<212>  DNA
<213>  Homo sapiens

<400>  48
agagtttgtt cttcgagtga tgactcagtt gactctggcc ctgaaggaat gccacagacg      60

aagtgatggt ggtcataccg tattgcatcg ggatctgaaa                            100


<210>  49
<211>  100
<212>  DNA
<213>  Homo sapiens

<400>  49
agatacggtc cagaagctta aaaatgccag acaagaagtg gtggagaaat atgaaatcta      60

tggagactca gttgactgcc tgccttcatg tcagttggaa                            100


<210>  50
<211>  100
<212>  DNA
<213>  Homo sapiens

<400>  50
agtgctaacg cacaatcgct taaaatcact aatgaagatt ctgagtgaag tgactcctga      60

ccagtccaag ccagaaaact gatcctgtac caaagcttga                          100

<210>  51
<211>  100
<212>  DNA
<213>  Homo sapiens

<400>  51
gctgatgtgt ttatcaaatg ataactggaa gctgaggaga atatgcctca aaaagagtag      60

ctccttggat acttcagact ctggttacag attgtcttga                          100

<210>  52
<211>  100
<212>  DNA
<213>  Homo sapiens

<400>  52
ctacacagtt aactagtgga gctgtgtggg ttcagtttaa tgatgggtcc cagttggttg      60

tgcaggcagg agtgtcttct atcagttata cctcaccaaa                          100

<210>  53
<211>  100
<212>  DNA
<213>  Homo sapiens

<400>  53
acagaaagtc tgctccagct ccacgatgct gagattgtgc ggttaaaaaa ctactatgaa      60

gttcacaagg aactctttga aggtgtccag aagtgggaag                          100

<210>  54
<211>  100
<212>  DNA
<213>  Homo sapiens

<400>  54
tgtgaagatg ccctctccac catgggaatc caatctgttg cagtctcctt caagcattct      60

gtcgaccctg gatgttgaat tgccacctgt ttgctgtgac                          100

<210>  55
<211>  100
<212>  DNA
<213>  Homo sapiens

<400>  55
tctgtgaagt ctcccaatca gagtctccgc cttggcttgt ccagattagc acgagttaaa      60

cctttgcatc caaatgccac tagcacctga gtgtggtaca                          100

<210>  56
<211>  100
<212>  DNA
<213>  Homo sapiens

<400>  56
agatctggag gcgctgaaag tggcagactc aaagacccta ttggagagcg agcgggaaag      60

gctgtttgcg aaaatggagg acacggactt tgtcggctgg                            100


<210>  57
<211>  100
<212>  DNA
<213>  Homo sapiens

<400>  57
aggggtgacc ctttagtgag cttagcacag cgggattaaa cagtccttta accagcacag      60

ccagttaaaa gatgcagcct cactgcttca acgcagattt                            100


<210>  58
<211>  100
<212>  DNA
<213>  Homo sapiens

<400>  58
agacctccat agatccttct ccagagagcc atgaagtaat ggaaagaata cttgacagcg      60

ttcagggaaa gtctactgta tctgaacaag ctgataagga                            100


<210>  59
<211>  100
<212>  DNA
<213>  Homo sapiens

<400>  59
aaggacttgt ggcttctctg attagtgtga aagacacttc tgcaaccaca gctttagaat      60

tagtggctgg agaacgactc tacaatgttg tagtagacac                            100


<210>  60
<211>  100
<212>  DNA
<213>  Homo sapiens

<400>  60
aacaagacat gatatttgag gcccgtttag ataccatggc agagacaaac agcatatctt      60

taaatggacc tttgagaaca gacgatctgg tgagagagga                            100


<210>  61
<211>  100
<212>  DNA
<213>  Homo sapiens

<400>  61
aatcagatca gcaggcaaaa taagctcatt caagaaaaaa aggataactt gttaaaattg      60

attgctgaag taaaaggcaa aaagcaggaa ttggaagtac                            100


<210>  62
<211>  100

<212>  DNA
<213>  Homo sapiens

<400>  62
tttaaccaag gaaaagaaag atgaactctg caggctaaga aatgaaaaag aacaagagct      60

ggacacatta aaaagaaaga gtccatcaga tttgtggaaa                            100


<210>  63
<211>  100
<212>  DNA
<213>  Homo sapiens

<400>  63
tgggacctgc tcttaacctc aaacctagga ccgtcttgct ttgtcattgg gcatggagag      60

aacccatttc tccagacttt tacctacccg tgcctgagaa                            100


<210>  64
<211>  100
<212>  DNA
<213>  Homo sapiens

<400>  64
aagagacaga aaacataatt gcagcaaatc actgggttct acctgcagct gaattccatg      60

ggctttggga cagcttggta tacgatgtgg aagtcaaatc                            100


<210>  65
<211>  100
<212>  DNA
<213>  Homo sapiens

<400>  65
aaccagaggt tccagagagt aaccagaaac agtggcaatc taagagaaag tcagagtgta      60

ttaaccagaa tcctgctgca tcttcaaatc actggcagat                            100


<210>  66
<211>  100
<212>  DNA
<213>  Homo sapiens

<400>  66
cttttaagaa gtacctgcaa gaaacctact caaagcaggt caccagccag gagccctgac      60

ccaggctgcc cagcctgtcc ttgtgtcgtc tttttaattt                            100


<210>  67
<211>  100
<212>  DNA
<213>  Homo sapiens

<400>  67
aaagactgtg aactcctgag ttcagcttga tttctgacta catcccagca agctctggca      60

tctgtggatt aaaatccctg gatctctctc agttgtgtat                            100

<210>  68
<211>  100
<212>  DNA
<213>  Homo sapiens

<400>  68
tgcagaagga gatcactgcc ctggcaccca gcacaatgaa gatcaagatc attgctcctc      60

ctgagcgcaa gtactccgtg tggatcggcg gctccatcct                            100


<210>  69
<211>  100
<212>  DNA
<213>  Homo sapiens

<400>  69
cgggcattcc tgaagctgac agcattcggg ccgagatgtc tcgctccgtg gccttagctg      60

tgctcgcgct actctctctt tctggcctgg aggctatcca                            100


<210>  70
<211>  100
<212>  DNA
<213>  Homo sapiens

<400>  70
cactcctcca cctttgacgc tggggctggc attgccctca acgaccactt tgtcaagctc      60

atttcctggt atgacaacga atttggctac agcaacaggg                            100


<210>  71
<211>  100
<212>  DNA
<213>  Homo sapiens

<400>  71
tgtgatgaag gagatgggag gccatcacat tgtagccctc tgtgtgctca agggggcta      60

taaattcttt gctgacctgc tggattacat caaagcactg                            100


<210>  72
<211>  100
<212>  DNA
<213>  Homo sapiens

<400>  72
gcaagaagta tgctgaggct gtcactcggg ctaagcagat tgtgtggaat ggtcctgtgg      60

gggtatttga atgggaagct tttgcccggg gaaccaaagc                            100


<210>  73
<211>  100
<212>  DNA
<213>  Homo sapiens

<400>  73
agcactggag agaaaggatt tggttataag ggttcctgct ttcacagaat tattccaggg      60

tttatgtgtc agggtggtga cttcacacgc cataatggca                    100

<210>  74
<211>  100
<212>  DNA
<213>  Homo sapiens

<400>  74
cgaaatgttt cattgtggga gcagacaatg tgggctccaa gcagatgcag cagatccgca      60

tgtcccttcg cgggaaggct gtggtgctga tgggcaagaa                    100

<210>  75
<211>  100
<212>  DNA
<213>  Homo sapiens

<400>  75
tggaggggca ggcttgcgag ctgcatttgg cctttctgag gcagggttta atacagcatg      60

tgttaccaag ctgtttccta ccaggtcaca cactgttgca                    100